(19) Europäisches Patentamt
European Patent Office
Office européen des brevets

(11) EP 0 675 681 B1

(12) **EUROPEAN PATENT SPECIFICATION**

(45) Date of publication and mention of the grant of the patent: **15.04.1998 Bulletin 1998/16**

(21) Application number: **94900960.9**

(22) Date of filing: **02.12.1993**

(51) Int. Cl.$^6$: **A01N 37/52**, C07C 259/12, C07C 259/14, C07D 333/54, C07D 213/75, C07D 239/47, C07C 323/41

(86) International application number: **PCT/GB93/02477**

(87) International publication number: **WO 94/14322 (07.07.1994 Gazette 1994/15)**

# (54) ARYLAMINO-ALKOXIMINO DERIVATIVES AND THEIR USE AS FUNGICIDES

ARYLAMINO-ALKOXIMINO DERIVATE UND DEREN VERWENDUNG ALS FUNGIZIDE

DERIVES ARYLAMINO-ALCOXIMINO ET LEUR UTILISATION COMME FONGICIDES

(84) Designated Contracting States: **CH DE DK FR GB LI**

(30) Priority: **22.12.1992 GB 9226734**

(43) Date of publication of application: **11.10.1995 Bulletin 1995/41**

(73) Proprietor: **ZENECA LIMITED**
**London W1Y 6LN (GB)**

(72) Inventors:
- **WORTHINGTON, Paul Anthony,**
  **4 Oakhurst**
  **Maidenhead, Berkshire SL6 8HY (GB)**
- **ASPINALL, Ian Henry,**
  **9 Barn Close**
  **Bracknell, Berkshire RG12 2TR (GB)**

(74) Representative:
**Tierney, Francis John et al**
**Intellectual Property Department,**
**ZENECA Agrochemicals,**
**Jealotts Hill Research Station,**
**P.O. Box 3538**
**Bracknell, Berkshire RG42 6YA (GB)**

(56) References cited:
**EP-A- 0 370 629**
**EP-A- 0 414 153**
**EP-A- 0 463 488**
**EP-A- 0 472 300**
**WO-A-92/13830**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 675 681 B1

## Description

The present invention relates to oxime derivatives that are useful as fungicides, to a process for preparing them, to compositions containing them and to methods of using them to combat fungi, especially fungal infections of plants.

The document EP-A1-0463488 discloses certain amino-alkoximino derivatives and their use as plant fungicides.

According to the present invention there is provided a compound having the general formula (I), wherein $R^1$ is hydrogen, $C_{1-4}$ alkyl, $C_{1-4}$ haloalkyl, $C_{2-4}$ alkenyl, $C_{2-4}$ alkynyl, $C_{3-6}$ cycloalkyl, aryl or aryl($C_{1-4}$)alkyl; $R^2$ is hydrogen, $C_{1-4}$ alkyl, $C_{1-4}$ haloalkyl or $C_{3-6}$ cycloalkyl; W is $CH_3O.CH{=}CCO_2CH_3$, $CH_3O.N{=}CCONR^3R^4$, $CH_3O.CH{=}CCONR^3R^4$ or $CH_3O.N{=}CCO_2CH_3$ and stereoisomers thereof; and $R^5$ is a phenyl or heterocyclyl moiety optionally substituted by halogen, hydroxy, mercapto, $C_{1-4}$ alkyl (especially methyl and ethyl), $C_{2-4}$ alkenyl (especially allyl), $C_{2-4}$ alkynyl (especially propargyl), $C_{1-4}$ alkoxy (especially methoxy), $C_{2-4}$ alkenyloxy (especially allyloxy), $C_{2-4}$ alkynyloxy (especially propargyloxy), halo($C_{1-4}$)alkyl (especially trifluoromethyl), halo($C_{1-4}$)-alkoxy (especially trifluoromethoxy), $C_{1-4}$ alkylthio (especially methylthio), hydroxy($C_{1-4}$)alkyl, $C_{1-4}$ alkoxy($C_{1-4}$)alkyl, $C_{3-6}$ cycloalkyl, $C_{3-6}$ cycloalkyl($C_{1-4}$)alkyl, optionally substituted methylenedioxy (especially optionally substituted with fluorine or $C_{1-4}$ alkyl), optionally substituted aryl (especially optionally substituted phenyl), optionally substituted heteroaryl (especially optionally substituted pyridyl or pyrimidinyl), optionally substituted heterocyclyl (especially optionally substituted pyrrolidine), optionally substituted aryloxy (especially optionally substituted phenoxy), optionally substituted heteroaryloxy (especially optionally substituted pyridyloxy or pyrimidinyloxy), optionally substituted aryl($C_{1-4}$)alkyl (especially optionally substituted benzyl, optionally substituted phenethyl and optionally substituted phenyl n-propyl) in which the alkyl moiety is optionally substituted with hydroxy, optionally substituted heteroaryl($C_{1-4}$)alkyl (especially optionally substituted pyridyl- or pyrimidinyl-($C_{1-4}$)alkyl), optionally substituted aryl($C_{2-4}$)alkenyl (especially optionally substituted phenylethenyl), optionally substituted heteroaryl($C_{2-4}$)alkenyl (especially optionally substituted pyridylethenyl or pyrimidinylethenyl), optionally substituted aryl($C_{1-4}$)alkoxy (especially optionally substituted benzyloxy), optionally substituted heteroaryl($C_{1-4}$)alkoxy (especially optionally substituted pyridyl- or pyrimidinyl($C_{1-4}$)alkoxy), optionally substituted aryloxy($C_{1-4}$)alkyl (especially phenoxymethyl), optionally substituted heteroaryloxy($C_{1-4}$)alkyl (especially optionally substituted pyridyloxy- or pyrimidinyloxy($C_{1-4}$)alkyl). acyloxy (including $C_{1-4}$ alkanoyloxy (especially acetyloxy) and benzoyloxy), cyano, thiocyanato, nitro, -NR'R", -NHCOR', -NHCONR'R", -CONR'R", -COOR', $-OSO_2R'$, $-SO_2R'$, -COR', -CR'=NR" or -N=CR'R" in which R' and R" are independently hydrogen, $C_{1-4}$ alkyl, $C_{1-4}$ alkoxy, $C_{1-4}$ alkylthio, $C_{3-6}$ cycloalkyl, $C_{3-6}$ cycloalkyl($C_{1-4}$)alkyl, phenyl or benzyl, the phenyl and benzyl groups being optionally substituted with halogen, $C_{1-4}$ alkyl or $C_{1-4}$ alkoxy; when R' and R" are in CONR'R" they can together form a 5- or 6-membered heterocyclic ring (for example a pyrrole, imidazole, pyrrolidine, piperidine or morpholine ring); or two substituents, when they are in adjacent positions on the aryl or heteroaryl ring can join to form a fused alphatic ring (especially to form a fused 6-membered carbon aliphatic ring); and $R^3$ and $R^4$ are independently hydrogen or $C_{1-4}$ alkyl.

In one particular aspect the present invention provides a compound of formula (Ia), wherein $R^1$ is hydrogen, $C_{1-4}$ alkyl, $C_{1-4}$ haloalkyl, $C_{2-4}$ alkenyl, $C_{2-4}$ alkynyl, $C_{3-6}$ cycloalkyl, aryl or aryl($C_{1-4}$)alkyl; $R^2$ is hydrogen, $C_{1-4}$ alkyl, $C_{1-4}$ haloalkyl or $C_{3-6}$ cycloalkyl; W is $CH_3O.CH{=}CCO_2CH_3$, $CH_3O.N{=}CCONR^3R^4$, $CH_3O.CH{=}CCONR^3R^4$ or $CH_3O.N{=}CCO_2CH_3$ and stereoisomers thereof; X, Y and Z are independently hydrogen, halogen, hydroxy, mercapto, $C_{1-4}$ alkyl (especially methyl and ethyl), $C_{2-4}$ alkenyl (especially allyl), $C_{2-4}$ alkynyl (especially propargyl), $C_{1-4}$ alkoxy (especially methoxy), $C_{2-4}$ alkenyloxy (especially allyloxy), $C_{2-4}$ alkynyloxy (especially propargyloxy), halo($C_{1-4}$)alkyl (especially trifluoromethyl), halo($C_{1-4}$)alkoxy (especially trifluoromethoxy), $C_{1-4}$ alkylthio (especially methylthio), hydroxy($C_{1-4}$)alkyl, $C_{1-4}$ alkoxy($C_{1-4}$)alkyl, $C_{3-6}$ cycloalkyl, $C_{3-6}$ cycloalkyl($C_{1-4}$)alkyl, optionally substituted methylenedioxy (especially optionally substituted with fluorine or $C_{1-4}$ alkyl), optionally substituted aryl (especially optionally substituted phenyl), optionally substituted heteroaryl (especially optionally substituted pyridyl or pyrimidinyl), optionally substituted heterocyclyl (especially optionally substituted pyrrolidine), optionally substituted aryloxy (especially optionally substituted phenoxy), optionally substituted heteroaryloxy (especially optionally substituted pyridyloxy or pyrimidinyloxy), optionally substituted aryl($C_{1-4}$)alkyl (especially optionally substituted benzyl, optionally substituted phenethyl and optionally substituted phenyl n-propyl) in which the alkyl moiety is optionally substituted with hydroxy, optionally substituted heteroaryl($C_{1-4}$)alkyl (especially optionally substituted pyridyl- or pyrimidinyl($C_{1-4}$)alkyl), optionally substituted aryl($C_{2-4}$)alkenyl (especially optionally substituted phenylethenyl), optionally substituted heteroaryl-($C_{2-4}$)alkenyl (especially optionally substituted pyridylethenyl or pyrimidinylethenyl), optionally substituted aryl($C_{1-4}$)alkoxy (especially optionally substituted benzyloxy), optionally substituted heteroaryl($C_{1-4}$)alkoxy (especially optionally substituted pyridyl- or pyrimidinyl($C_{1-4}$)alkoxy), optionally substituted aryloxy($C_{1-4}$)alkyl (especially phenoxymethyl), optionally substituted heteroaryloxy($C_{1-4}$)alkyl (especially optionally substituted pyridyloxy- or pyrimidinyloxy($C_{1-4}$)alkyl), acyloxy (including $C_{1-4}$ alkanoyloxy (especially acetyloxy) and benzoyloxy), cyano, thiocyanato, nitro, -NR'R", -NHCOR', -NHCONR'R", -CONR'R", -COOR', $-OSO_2R'$, $-SO_2R'$, -COR', -CR'=NR" or -N=CR'R" in which R' and R" are independently hydrogen, $C_{1-4}$ alkyl, $C_{1-4}$ alkoxy, $C_{1-4}$ alkylthio, $C_{3-6}$ cycloalkyl, $C_{3-6}$ cycloalkyl($C_{1-4}$)alkyl, phenyl or benzyl, the phenyl and benzyl groups being optionally substituted with halogen, $C_{1-4}$ alkyl or $C_{1-4}$ alkoxy; when R' and R" are in CONR'R" they can together form a 5- or 6-membered heterocyclic ring (for example a pyrrole, imidazole, pyrrolidine,

piperidine or morpholine ring); or two substituents, when they are in adjacent positions on the aryl or heteroaryl ring can join to form a fused alphatic ring (especially to form a fused 6-membered carbon aliphatic ring); and $R^3$ and $R^4$ are independently hydrogen or $C_{1-4}$ alkyl.

Because the double bond of the propenoate, oxyiminoacetamide or oxyiminoacetate group is unsymmetrically substituted, the compounds of the invention may be obtained in the form of mixtures of (E)- and (Z)-geometric isomers. However, these mixtures can be separated into individual isomers, and this invention embraces such isomers and mixtures thereof in all proportions including those which consist substantially of the (Z)-isomer and those which consist substantially of the (E)-isomer. The (E)-isomers are the more fungicidally active and form a preferred embodiment of the invention.

Optionally substituted methylenedioxy includes $-OCH_2O-$, -OCHFO-, $-OCF_2O$, $-OCH(CH_3)O-$ and $-OC(CH_3)_2O-$.

Substituents which may be present in the aryl or heteroaryl rings of any of the foregoing substituents include one or more of the following: halo, hydroxy, mercapto, $C_{1-4}$ alkyl, $C_{2-4}$ alkenyl, $C_{2-4}$ alkynyl, $C_{1-4}$ alkoxy, $C_{2-4}$ alkenyloxy, $C_{2-4}$ alkynyloxy, halo($C_{1-4}$)alkyl, halo($C_{1-4}$)alkoxy, $C_{1-4}$ alkylthio, hydroxy($C_{1-4}$)alkyl, $C_{1-4}$ alkoxy($C_{1-4}$)alkyl, $C_{3-6}$ cycloalkyl, $C_{3-6}$ cycloalkyl($C_{1-4}$)alkyl, alkanoyloxy, benzoyloxy, cyano, thiocyanato, nitro, -NR'R", -NHCOR', -NHCONR'R", -CONR'R", -COOR', $-SO_2R'$, $-OSO_2R'$, -COR', -CR'=NR" or -N=CR'R" in which R' and R" have the meanings given above.

It is preferred that $R^3$ is hydrogen and $R^4$ is methyl.

Aryl is preferably phenyl.

Heteroaryl includes 5- and 6-membered aromatic rings containing one or more heteroatoms selected from the list comprising oxygen, sulphur and nitrogen and can be fused to benzenoid ring systems. Examples of heteroaryl are pyridinyl pyrimidinyl, pyridazinyl, pyrazinyl, triazinyl, furyl, thienyl, pyrrolyl, pyrazolyl, imidazolyl, triazolyl, oxazolyl, isoxazolyl, thiazolyl, isothiazolyl, quinolinyl, isoquinolinyl, cinnolinyl, quinazolinyl, quinoxalinyl, indolinyl, isoindolinyl, benzofuranyl, benzothiophenyl and benzimidazolinyl.

Heterocyclyl includes heteroaryl as previously defined and includes non-aromatic rings such as morpholine and piperidine.

Alkyl moieties and the alkyl moiety of alkoxy and haloalkyl preferably contain, unless otherwise stated, from 1 to 6, more preferably from 1 to 4, carbon atoms. They can be in the form of straight or branched chains, for example methyl, ethyl, n- or iso- propyl, or n-, sec-, iso- or tert-butyl.

Alkenyl and alkynyl moieties preferably contain, unless otherwise stated, from 2 to 6, more preferably from 2 to 4, carbon atoms. They can be in the form of straight or branched chains, and the alkenyl moieties, where appropriate, may have either the (E)- or (Z)-configuration. Examples are vinyl, allyl and propargyl.

Halogen is typically fluorine, chlorine or bromine.

In another aspect the present invention provides a compound of formula (I) or (Ia) wherein W is $CH_3O.CH=CCO_2CH_3$.

It is preferred that $R^1$ is hydrogen or $C_{1-4}$ alkyl (especially methyl). Compounds wherein $R^1$ is hydrogen form a preferred embodiment of the present invention.

It is preferred that $R^2$ is methyl.

In a further aspect the present invention provides a compound of formula (I) wherein $R^5$ is a phenyl substituted with at least one electron withdrawing group. Electron withdrawing groups are, for example, halogen (such as fluorine, chlorine or bromine), cyano, nitro, esters (such as $CO_2$alkyl) or halo($C_{1-4}$)alkyl (such as $CF_3$), and an electron withdrawing substituent may be combined with one or more other electron withdrawing substituents or with one or more other substituents on the phenyl ring.

In a still further aspect the present invention provides a compound of formula (Ib), wherein $R^1$ is hydrogen or $C_{1-4}$ alkyl (such as methyl); and $R^5$ is phenyl optionally substituted (such as unsubstituted or substituted with 1, 2 or 3 substituents) with halogen (such as fluorine or chlorine), $C_{1-4}$ alkyl (such as methyl), $C_{2-4}$ alkenyl (such as vinyl or allyl), $C_{2-4}$ alkynyl (such as propargyl), $C_{1-4}$ alkoxy (such as methoxy or ethoxy), $C_{2-4}$ alkenyloxy (such as allyloxy), $C_{2-4}$ alkynyloxy (such as propargyloxy), halo($C_{1-4}$)alkyl (such as $CF_3$), halo($C_{1-4}$)alkoxy (such as $OCF_3$), $C_{1-4}$ alkoxy($C_{1-4}$)alkyl (such as methoxymethyl), acyloxy (such as acetyloxy or benzoyloxy), cyano, thiocyanato, nitro, -NR'R", -NHCOR', -CONR'R", COOR' or COR' in which R' and R" are independently hydrogen, $C_{1-4}$ alkyl or $C_{1-4}$ alkoxy.

In another aspect the present invention provides a compound of formula (Ib), wherein $R^1$ is hydrogen, and $R^5$ is phenyl optionally substituted with halogen (especially chlorine or fluorine), cyano, nitro, halo($C_{1-4}$)alkyl (such as $CF_3$, $C_2F_5$, $CF_3CH_2$ or $CH_3CF_2$) or -COOR' in which R' is $C_{1-4}$ alkyl (especially methyl).

The present invention provides a compound of formula (Ia) wherein $R^1$ is hydrogen or $C_{1-4}$ alkyl (especially methyl); $R^2$ is $C_{1-4}$ alkyl (especially methyl); W is $CH_3O.CH=CCO_2CH_3$, $CH_3O.N=CCO_2CH_3$, $CH_3O.N=CCONHR^4$ or $CH_3O.CH=CCONHR^4$ (wherein $R^4$ is $C_{1-4}$ alkyl (especially methyl)); and Y and Z are both hydrogen and X is hydrogen, $C_{1-4}$ haloalkyl (especially trifluoromethyl), halogen (especially chlorine), $C_{1-4}$ alkyl (especially methyl), cyano or $C_{1-4}$ alkoxy (especially methoxy).

The present invention is illustrated by compounds of formula (I) which are listed in Tables 1 to 8. Compounds in

Table 1 are of general formula (I) wherein W is $CH_3O.CH{=}CCO_2CH_3$, $R^1$ is hydrogen and $R^2$ is methyl.

TABLE 1

| Compound No | $R^5$ | Compound No | $R^5$ |
|---|---|---|---|
| 1 | $C_6H_5$ | 32 | $3\text{-Br-}C_6H_4$ |
| 2 | $3\text{-}CF_3\text{-}C_6H_4$ | 33 | $4\text{-Br-}C_6H_4$ |
| 3 | $2\text{-}CF_3\text{-}C_6H_4$ | 34 | $2\text{-}NO_2\text{-}C_6H_4$ |
| 4 | $4\text{-}CF_3\text{-}C_6H_4$ | 35 | $3\text{-}NO_2\text{-}C_6H_4$ |
| 5 | $3\text{-Cl-}C_6H_4$ | 36 | $4\text{-}NO_2\text{-}C_6H_4$ |
| 6 | $2\text{-Cl-}C_6H_4$ | 37 | $2,3\text{-}Cl_2\text{-}C_6H_3$ |
| 7 | $4\text{-Cl-}C_6H_4$ | 38 | $2,4\text{-}Cl_2\text{-}C_6H_3$ |
| 8 | $2\text{-}CH_3\text{-}C_6H_4$ | 39 | $2,5\text{-}Cl_2\text{-}C_6H_3$ |
| 9 | $3\text{-}CH_3\text{-}C_6H_4$ | 40 | $2,6\text{-}Cl_2\text{-}C_6H_3$ |
| 10 | $4\text{-}CH_3\text{-}C_6H_4$ | 41 | $3,4\text{-}Cl_2\text{-}C_6H_3$ |
| 11 | $2\text{-CN-}C_6H_4$ | 42 | $3,5\text{-}Cl_2\text{-}C_6H_3$ |
| 12 | $3\text{-CN-}C_6H_4$ | 43 | $C_6Cl_5$ |
| 13 | $4\text{-CN-}C_6H_4$ | 44 | $2,3,4\text{-}Cl_3\text{-}C_6H_2$ |
| 14 | $2\text{-}CH_3O\text{-}C_6H_4$ | 45 | $2,4,6\text{-}Cl_3\text{-}C_6H_2$ |
| 15 | $3\text{-}CH_3O\text{-}C_6H_4$ | 46 | $3,4,5\text{-}Cl_3\text{-}C_6H_2$ |
| 16 | $4\text{-}CH_3O\text{-}C_6H_4$ | 47 | $2\text{-}C_2F_5\text{-}C_6H_4$ |
| 17 | $2\text{-F-}C_6H_4$ | 48 | $3\text{-}C_2H_5\text{-}C_6H_4$ |
| 18 | $3\text{-F-}C_6H_4$ | 49 | $4\text{-}C_2H_5\text{-}C_6H_4$ |
| 19 | $4\text{-F-}C_6H_4$ | 50 | $2\text{-}CCl_3\text{-}C_6H_4$ |
| 20 | $2,3\text{-}F_2\text{-}C_6H_3$ | 51 | $3\text{-}CCl_3\text{-}C_6H_4$ |
| 21 | $2,4\text{-}F_2\text{-}C_6H_3$ | 52 | $4\text{-}CCl_3\text{-}C_6H_4$ |
| 22 | $2,5\text{-}F_2\text{-}C_6H_3$ | 53 | $2\text{-}CO_2CH_3\text{-}C_6H_4$ |
| 23 | $2,6\text{-}F_2\text{-}C_6H_3$ | 54 | $3\text{-}CO_2CH_3\text{-}C_6H_4$ |
| 24 | $3,4\text{-}F_2\text{-}C_6H_3$ | 55 | $4\text{-}CO_2CH_3\text{-}C_6H_4$ |
| 25 | $3,5\text{-}F_2\text{-}C_6H_3$ | 56 | $2\text{-}CO_2C_2H_5\text{-}C_6H_4$ |
| 26 | $C_6F_5$ | 57 | $3\text{-}CO_2C_2H_5\text{-}C_6H_4$ |
| 27 | $2,3,4\text{-}F_3\text{-}C_6H_2$ | 58 | $4\text{-}CO_2C_2H_5\text{-}C_6H_4$ |
| 28 | $2,3,5\text{-}F_3\text{-}C_6H_2$ | 59 | $2\text{-}CO_2((CH_2)_2CH_3)\text{-}C_6H_4$ |
| 29 | $2,4,6\text{-}F_3\text{-}C_6H_2$ | 60 | $3\text{-}CO_2((CH_2)_2CH_3)\text{-}C_6H_4$ |
| 30 | $3,4,5\text{-}F_3\text{-}C_6H_2$ | 61 | $4\text{-}CO_2((CH_2)_2CH_3)\text{-}C_6H_4$ |
| 31 | $2\text{-Br-}C_6H_4$ | 62 | $2\text{-}CO_2((CH_3)_2CH)\text{-}C_6H_4$ |

| Compound No | $R^5$ | Compound No | $R^5$ |
|---|---|---|---|
| 63 | 3-$CO_2((CH_3)_2CH)$-$C_6H_4$ | 93 | 4-Methylpyridin-3-yl |
| 64 | 4-$CO_2((CH_3)_2CH)$-$C_6H_4$ | 94 | 5-Methylpyridin-3-yl |
| 65 | 2-F-3-Cl-$C_6H_3$ | 95 | 6-Methylpyridin-3-yl |
| 66 | 2-Cl-3-F-$C_6H_3$ | 96 | 2-Methylpyridin-4-yl |
| 67 | 2-F-4-Cl-$C_6H_3$ | 97 | 3-Methylpyridin-4-yl |
| 68 | 2-Cl-4-F-$C_6H_3$ | 98 | 4-Cyclopropyl-pyrimidin-2-yl |
| 69 | 2-F-5-Cl-$C_6H_3$ | | |
| 70 | 2-Cl-5-F-$C_6H_3$ | 99 | 4-Methyl-6-methoxy-pyrimidin-2-yl |
| 71 | 2-F-6-Cl-$C_6H_3$ | | |
| 72 | 3-F-5-Cl-$C_6H_3$ | 100 | 5-Chloropyrimidin-2-yl |
| 73 | 3-F-4-Cl-$C_6H_3$ | 101 | 4,6-Dimethyl-pyrimidin-2-yl |
| 74 | 3-Cl-4-Cl-$C_6H_3$ | | |
| 75 | 2-$CF_3CH_2$-$C_6H_4$ | 102 | 2-$CH_3OCH_2$-$C_6H_4$ |
| 76 | 3-$CF_3CH_2$-$C_6H_4$ | 103 | 3-$CH_3OCH_2$-$C_6H_4$ |
| 77 | 4-$CF_3CH_2$-$C_6H_4$ | 104 | 4-$CH_3OCH_2$-$C_6H_4$ |
| 78 | 2-$CH_3CF_2$-$C_6H_4$ | 105 | 2-$NH_2$-$C_6H_4$ |
| 79 | 3-$CH_3CF_2$-$C_6H_4$ | 106 | 3-$NH_2$-$C_6H_4$ |
| 80 | 4-$CH_3CF_2$-$C_6H_4$ | 107 | 4-$NH_2$-$C_6H_4$ |
| 81 | 2-$CF_3(CF_2)_2$-$C_6H_4$ | 108 | 2,3-$(CH_3)_2$-$C_6H_3$ |
| 82 | 3-$CF_3(CF_2)_2$-$C_6H_4$ | 109 | 2,4-$(CH_3)_2$-$C_6H_3$ |
| 83 | 4-$CF_3(CF_2)_2$-$C_6H_4$ | 110 | 2,5-$(CH_3)_2$-$C_6H_3$ |
| 84 | 2-$(CF_3)_2CF$-$C_6H_4$ | 111 | 2,6-$(CH_3)_2$-$C_6H_3$ |
| 85 | 3-$(CF_3)_2CF$-$C_6H_4$ | 112 | 2-$CH_3$-3-$NO_2$-$C_6H_3$ |
| 86 | 4-$(CF_3)_2CF$-$C_6H_4$ | 113 | 2-$CH_3$-4-$NO_2$-$C_6H_3$ |
| 87 | 3-Methylbenzo[b]-thiophen-7-yl | 114 | 2-$CH_3$-5-$NO_2$-$C_6H_3$ |
| | | 115 | 2-$CH_3$-6-$NO_2$-$C_6H_3$ |
| 88 | 3-Methylpyridin-2-yl | 116 | 3-$CH_3$-2-$NO_2$-$C_6H_3$ |
| 89 | 4-Methylpyridin-2-yl | 117 | 3-$CH_3$-4-$NO_2$-$C_6H_3$ |
| 90 | 5-Methylpyridin-2-yl | 118 | 3-$CH_3$-5-$NO_2$-$C_6H_3$ |
| 91 | 6-Methylpyridin-2-yl | 119 | 3-$CH_3$-6-$NO_2$-$C_6H_3$ |
| 92 | 2-Methylpyridin-3-yl | | |

| Compound No | $R^5$ | Compound No | $R^5$ |
|---|---|---|---|
| 120 | $2-NO_2-4-CH_3-C_6H_3$ | 151 | $4-F-3-CF_3-C_6H_3$ |
| 121 | $3-NO_2-4-CH_3-C_6H_3$ | 152 | $2-CH_3O-3-NO_2-C_6H_3$ |
| 122 | $2-CH_3-3-CN-C_6H_3$ | 153 | $2-CH_3O-4-NO_2-C_6H_3$ |
| 123 | $2-CH_3-4-CN-C_6H_3$ | 154 | $2-CH_3O-5-NO_2-C_6H_3$ |
| 124 | $2-CH_3-5-CN-C_6H_3$ | 155 | $2-CH_3O-6-NO_2-C_6H_3$ |
| 125 | $2-CH_3-6-CN-C_6H_3$ | 156 | $3-CH_3O-2-NO_2-C_6H_3$ |
| 126 | $3-CH_3-2-CN-C_6H_3$ | 157 | $3-CH_3O-4-NO_2-C_6H_3$ |
| 127 | $3-CH_3-4-CN-C_6H_3$ | 158 | $3-CH_3O-5-NO_2-C_6H_3$ |
| 128 | $3-CH_3-5-CN-C_6H_3$ | 159 | $3-CH_3O-6-NO_2-C_6H_3$ |
| 129 | $3-CH_3-6-CN-C_6H_3$ | 160 | $4-CH_3O-2-NO_2-C_6H_3$ |
| 130 | $4-CH_3-2-CN-C_6H_3$ | 161 | $4-CH_3O-3-NO_2-C_6H_3$ |
| 131 | $4-CH_3-3-CN-C_6H_3$ | 162 | $2-CH_3O-3-CF_3-C_6H_3$ |
| 132 | $2-F-3-NO_2-C_6H_3$ | 163 | $2-CH_3O-4-CF_3-C_6H_3$ |
| 133 | $2-F-4-NO_2-C_6H_3$ | 164 | $2-CH_3O-5-CF_3-C_6H_3$ |
| 134 | $2-F-5-NO_2-C_6H_3$ | 165 | $2-CH_3O-6-CF_3-C_6H_3$ |
| 135 | $2-F-6-NO_2-C_6H_3$ | 166 | $3-CH_3O-2-CF_3-C_6H_3$ |
| 136 | $3-F-2-NO_2-C_6H_3$ | 167 | $3-CH_3O-4-CF_3-C_6H_3$ |
| 137 | $3-F-4-NO_2-C_6H_3$ | 168 | $3-CH_3O-5-CF_3-C_6H_3$ |
| 138 | $3-F-5-NO_2-C_6H_3$ | 169 | $3-CH_3O-6-CF_3-C_6H_3$ |
| 139 | $3-F-6-NO_2-C_6H_3$ | 170 | $4-CH_3O-2-CF_3-C_6H_3$ |
| 140 | $4-F-2-NO_2-C_6H_3$ | 171 | $4-CH_3O-3-CF_3-C_6H_3$ |
| 141 | $4-F-3-NO_2-C_6H_3$ | 172 | $2-CH_3-3-CH_3O-C_6H_3$ |
| 142 | $2-F-3-CF_3-C_6H_3$ | 173 | $2-CH_3-4-CH_3O-C_6H_3$ |
| 143 | $2-F-4-CF_3-C_6H_3$ | 174 | $2-CH_3-5-CH_3O-C_6H_3$ |
| 144 | $2-F-5-CF_3-C_6H_3$ | 175 | $2-CH_3-6-CH_3O-C_6H_3$ |
| 145 | $2-F-6-CF_3-C_6H_3$ | 176 | $3-CH_3-3-CH_3O-C_6H_3$ |
| 146 | $3-F-2-CF_3-C_6H_3$ | 177 | $3-CH_3-4-CH_3O-C_6H_3$ |
| 147 | $3-F-4-CF_3-C_6H_3$ | 178 | $3-CH_3-5-CH_3O-C_6H_3$ |
| 148 | $3-F-5-CF_3-C_6H_3$ | 179 | $3-CH_3-6-CH_3O-C_6H_3$ |
| 149 | $3-F-6-CF_3-C_6H_3$ | 180 | $4-CH_3-2-CH_3O-C_6H_3$ |
| 150 | $4-F-2-CF_3-C_6H_3$ | 181 | $4-CH_3-3-CH_3O-C_6H_3$ |

| Compound No | $R^5$ | Compound No | $R^5$ |
|---|---|---|---|
| 182 | 2-F-3-$CH_3$-$C_6H_3$ | 214 | 2-CN-5-F-$C_6H_3$ |
| 183 | 2-F-4-$CH_3$-$C_6H_3$ | 215 | 2-CN-6-F-$C_6H_3$ |
| 184 | 2-F-5-$CH_3$-$C_6H_3$ | 216 | 3-CN-2-F-$C_6H_3$ |
| 185 | 2-F-6-$CH_3$-$C_6H_3$ | 217 | 3-CN-4-F-$C_6H_3$ |
| 186 | 3-F-2-$CH_3$-$C_6H_3$ | 218 | 3-CN-5-F-$C_6H_3$ |
| 187 | 3-F-4-$CH_3$-$C_6H_3$ | 219 | 3-CN-6-F-$C_6H_3$ |
| 188 | 3-F-5-$CH_3$-$C_6H_3$ | 220 | 4-CN-2-F-$C_6H_3$ |
| 189 | 3-F-6-$CH_3$-$C_6H_3$ | 221 | 4-CN-3-F-$C_6H_3$ |
| 190 | 4-F-2-$CH_3$-$C_6H_3$ | 222 | 2-Cl-3-$CH_3O$-$C_6H_3$ |
| 191 | 4-F-3-$CH_3$-$C_6H_3$ | 223 | 2-Cl-4-$CH_3O$-$C_6H_3$ |
| 192 | 2-F-3-$CH_3O$-$C_6H_3$ | 224 | 2-Cl-5-$CH_3O$-$C_6H_3$ |
| 193 | 2-F-4-$CH_3O$-$C_6H_3$ | 225 | 2-Cl-6-$CH_3O$-$C_6H_3$ |
| 194 | 2-F-5-$CH_3O$-$C_6H_3$ | 226 | 3-Cl-2-$CH_3O$-$C_6H_3$ |
| 195 | 2-F-6-$CH_3O$-$C_6H_3$ | 227 | 3-Cl-4-$CH_3O$-$C_6H_3$ |
| 196 | 3-F-2-$CH_3O$-$C_6H_3$ | 228 | 3-Cl-5-$CH_3O$-$C_6H_3$ |
| 197 | 3-F-4-$CH_3O$-$C_6H_3$ | 229 | 3-Cl-6-$CH_3O$-$C_6H_3$ |
| 198 | 3-F-5-$CH_3O$-$C_6H_3$ | 230 | 4-Cl-2-$CH_3O$-$C_6H_3$ |
| 199 | 3-F-6-$CH_3O$-$C_6H_3$ | 231 | 4-Cl-3-$CH_3O$-$C_6H_3$ |
| 200 | 4-F-2-$CH_3O$-$C_6H_3$ | 232 | 3,4-$OCH_2O$-$C_6H_3$ |
| 201 | 4-F-3-$CH_3O$-$C_6H_3$ | 233 | 3,4-$OC_2H_4O$-$C_6H_3$ |
| 202 | 2-CN-3-$CH_3O$-$C_6H_3$ | 234 | 4-F-2-pyridinyl |
| 203 | 2-CN-4-$CH_3O$-$C_6H_3$ | 235 | 4-Cl-2-pyridinyl |
| 204 | 2-CN-5-$CH_3O$-$C_6H_3$ | 236 | 2-F-4-pyridinyl |
| 205 | 2-CN-6-$CH_3O$-$C_6H_3$ | 237 | 2-Cl-4-pyridinyl |
| 206 | 3-CN-2-$CH_3O$-$C_6H_3$ | 238 | 2-Cl-6-pyridinyl |
| 207 | 3-CN-4-$CH_3O$-$C_6H_3$ | 239 | 2-F-6-pyridinyl |
| 208 | 3-CN-5-$CH_3O$-$C_6H_3$ | 240 | 2-$CF_3$-6-pyridinyl |
| 209 | 3-CN-6-$CH_3O$-$C_6H_3$ | 241 | 4-$CF_3$-2-pyridinyl |
| 210 | 4-CN-2-$CH_3O$-$C_6H_3$ | 242 | 2-$CH_3O$-3-pyridinyl |
| 211 | 4-CN-3-$CH_3O$-$C_6H_3$ | 243 | 2-$CH_3O$-4-pyridinyl |
| 212 | 2-CN-3-F-$C_6H_3$ | 244 | 2-$CH_3O$-5-pyridinyl |
| 213 | 2-CN-4-F-$C_6H_3$ | 245 | 2-$CH_3O$-6-pyridinyl |

| Compound No | $R^5$ | Compound No | $R^5$ |
|---|---|---|---|
| 246 | 3-$CH_3$O-2-pyridinyl | 280 | 3-Cl-5-$CH_3$-$C_6H_3$ |
| 247 | 3-$CH_3$O-4-pyridinyl | 281 | 3-Cl-6-$CH_3$-$C_6H_3$ |
| 248 | 3-$CH_3$O-5-pyridinyl | 282 | 4-Cl-2-$CH_3$-$C_6H_3$ |
| 249 | 3-$CH_3$O-6-pyridinyl | 283 | 4-Cl-3-$CH_3$-$C_6H_3$ |
| 250 | 4-$CH_3$O-2-pyridinyl | 284 | 2-Cl-3-$NO_2$-$C_6H_3$ |
| 252 | 4-$CH_3$O-3-pyridinyl | 285 | 2-Cl-4-$NO_2$-$C_6H_3$ |
| 253 | 2,6-diF-3-pyridinyl | 286 | 2-Cl-5-$NO_2$-$C_6H_3$ |
| 254 | 5-Cl-2-pyrimidinyl | 287 | 2-Cl-6-$NO_2$-$C_6H_3$ |
| 255 | 5-F-2-pyrimidinyl | 288 | 3-Cl-2-$NO_2$-$C_6H_3$ |
| 256 | 5-$CH_3$-2-pyrimidinyl | 289 | 3-Cl-4-$NO_2$-$C_6H_3$ |
| 257 | 4-$CH_3$O-2-pyrimidinyl | 290 | 3-Cl-5-$NO_2$-$C_6H_3$ |
| 258 | 2-$CH_3$O-4-pyrimidinyl | 291 | 3-Cl-6-$NO_2$-$C_6H_3$ |
| 259 | 2-$CH_3$O-5-pyrimidinyl | 292 | 4-Cl-2-$NO_2$-$C_6H_3$ |
| 260 | 4-$CH_3$O-6-pyrimidinyl | 293 | 4-Cl-3-$NO_2$-$C_6H_3$ |
| 261 | 5-$CH_3$O-4-pyrimidinyl | 294 | 2-Cl-3-$CF_3$-$C_6H_3$ |
| 262 | 6-$CH_3$O-5-pyrimidinyl | 295 | 2-Cl-4-$CF_3$-$C_6H_3$ |
| 263 | 4-$C_2H_5$O-2-pyrimidinyl | 296 | 2-Cl-5-$CF_3$-$C_6H_3$ |
| 264 | 2-$CF_3$-4-pyrimidinyl | 297 | 2-Cl-6-$CF_3$-$C_6H_3$ |
| 265 | 4-$CF_3$-2-pyrimidinyl | 298 | 3-Cl-2-$CF_3$-$C_6H_3$ |
| 266 | 4-$CF_3$-5-pyrimidinyl | 299 | 3-Cl-4-$CF_3$-$C_6H_3$ |
| 267 | 4-$CF_3$-6-pyrimidinyl | 200 | 3-Cl-5-$CF_3$-$C_6H_3$ |
| 268 | 5-$CF_3$-6-pyrimidinyl | 301 | 3-Cl-6-$CF_3$-$C_6H_3$ |
| 269 | 4-$CF_3CH_2$O-2-pyrimidinyl | 302 | 4-Cl-2-$CF_3$-$C_6H_3$ |
| 270 | 6-$CH_3$O-2-benzthiazolyl | 303 | 4-Cl-3-$CF_3$-$C_6H_3$ |
| 271 | 2-$CH_3$S-$C_6H_4$ | 304 | 2-Cl-3-Br-$C_6H_3$ |
| 272 | 3-$CH_3$S-$C_6H_4$ | 305 | 2-Cl-4-Br-$C_6H_3$ |
| 273 | 4-$CH_3$S-$C_6H_4$ | 306 | 2-Cl-5-Br-$C_6H_3$ |
| 274 | 2-Cl-3-$CH_3$-$C_6H_3$ | 307 | 2-Cl-6-Br-$C_6H_3$ |
| 275 | 2-Cl-4-$CH_3$-$C_6H_3$ | 308 | 3-Cl-2-Br-$C_6H_3$ |
| 276 | 2-Cl-5-$CH_3$-$C_6H_3$ | 309 | 3-Cl-4-Br-$C_6H_3$ |
| 277 | 2-Cl-6-$CH_3$-$C_6H_3$ | 310 | 3-Cl-5-Br-$C_6H_3$ |
| 278 | 3-Cl-2-$CH_3$-$C_6H_3$ | 311 | 3-Cl-6-Br-$C_6H_3$ |
| 279 | 3-Cl-4-$CH_3$-$C_6H_3$ | 312 | 4-Cl-2-Br-$C_6H_3$ |

| Compound No | $R^5$ | Compound No | $R^5$ |
|---|---|---|---|
| 313 | $4-Cl-3-Br-C_6H_3$ | 337 | $2,4-(OCH_3)_2-C_6H_3$ |
| 314 | $2-Cl-3-CN-C_6H_3$ | 338 | $2,5-(OCH_3)_2-C_6H_3$ |
| 315 | $2-Cl-4-CN-C_6H_3$ | 339 | $2,6-(OCH_3)_2-C_6H_3$ |
| 316 | $2-Cl-5-CN-C_6H_3$ | 340 | $3,4-(OCH_3)_2-C_6H_3$ |
| 317 | $2-Cl-6-CN-C_6H_3$ | 341 | $3,5-(OCH_3)_2-C_6H_3$ |
| 318 | $3-Cl-2-CN-C_6H_3$ | 342 | $2,3-(CN)_2-C_6H_3$ |
| 319 | $3-Cl-4-CN-C_6H_3$ | 346 | $2,4-(CN)_2-C_6H_3$ |
| 320 | $3-Cl-5-CN-C_6H_3$ | 347 | $2,5-(CN)_2-C_6H_3$ |
| 321 | $3-Cl-6-CN-C_6H_3$ | 348 | $2,6-(CN)_2-C_6H_3$ |
| 322 | $4-Cl-2-CN-C_6H_3$ | 349 | $3,4-(CN)_2-C_6H_3$ |
| 323 | $4-Cl-3-CN-C_6H_3$ | 350 | $3,5-(CN)_2-C_6H_3$ |
| 324 | $2,3-(NO_2)_2-C_6H_3$ | 351 | $2,3-(OCF_3)_2-C_6H_3$ |
| 325 | $2,4-(NO_2)_2-C_6H_3$ | 352 | $2,4-(OCF_3)_2-C_6H_3$ |
| 326 | $2,5-(NO_2)_2-C_6H_3$ | 353 | $2,5-(OCF_3)_2-C_6H_3$ |
| 327 | $2,6-(NO_2)_2-C_6H_3$ | 354 | $2,6-(OCF_3)_2-C_6H_3$ |
| 328 | $3,4-(NO_2)_2-C_6H_3$ | 355 | $3,4-(OCF_3)_2-C_6H_3$ |
| 329 | $3,5-(NO_2)_2-C_6H_3$ | 356 | $3,5-(OCF_3)_2-C_6H_3$ |
| 330 | $2,3-(CF_3)_2-C_6H_3$ | 357 | $2,3,4,5-F_4-C_6H$ |
| 331 | $2,4-(CF_3)_2-C_6H_3$ | 358 | $2,3,5,6-F_4-C_6H$ |
| 332 | $2,5-(CF_3)_2-C_6H_3$ | 359 | $2,3,4,6-F_4-C_6H$ |
| 333 | $2,6-(CF_3)_2-C_6H_3$ | 360 | $2,3,4,5-Cl_4-C_6H$ |
| 334 | $3,4-(CF_3)_2-C_6H_3$ | 361 | $2,3,4,5-Cl_4-C_6H$ |
| 335 | $3,5-(CF_3)_2-C_6H_3$ | 362 | $2,3,4,5-Cl_4-C_6H$ |
| 336 | $2,3-(OCH_3)_2-C_6H_3$ | | |

TABLE 2

Table 2 comprises 362 compounds of formula (I) wherein W is $CH_3O.CH{=}CCO_2CH_3$, $R^1$ and $R^2$ are both methyl and $R^5$ is as listed for Table 1.

TABLE 3

Table 3 comprises 362 compounds of formula (I) wherein W is $CH_3O.CH{=}CCONHCH_3$; $R^1$ is hydrogen; $R^2$ is methyl, and $R^5$ is as listed for Table 1.

TABLE 4

Table 4 comprises 362 compounds of formula (I) wherein W is $CH_3O.CH{=}CCONHCH_3$; $R^1$ and $R^2$ are both methyl; and $R^5$ is as listed for Table 1.

TABLE 5

Table 5 comprises 362 compounds of formula (I) wherein W is $CH_3O.N{=}CCO_2CH_3$; $R^1$ is hydrogen; $R^2$ is methyl, and $R^5$ is as listed for Table I.

TABLE 6

Table 6 comprises 362 compounds of formula (I) wherein W is $CH_3O.N{=}CCO_2CH_3$; $R^1$ and $R^2$ are both methyl and $R^5$ is as listed for Table I.

TABLE 7

Table 7 comprises 362 comopunds of formula (I) wherein W is $CH_3O.N{=}CCONHCH_3$; $R^1$ is hydrogen; $R^2$ is methyl and $R^5$ is as listed for Table I.

TABLE 8

Table 8 comprises 362 compounds of formula (I) wherein W is $CH_3O.N{=}CCONHCH_3$; $R^1$ and $R^2$ are both methyl and $R^5$ is as listed for Table I.

TABLE I

Table I gives melting points and proton NMR data obtained at 270 MHz for certain compounds described in Tables 1 to 8. Chemical shifts are measured at 20°C in ppm from tetramethylsilane and deuterochloroform was used as solvent, unless otherwise stated. The following abbreviations are used:

| | | | | | |
|---|---|---|---|---|---|
| s | = | singlet | m | = | multiplet |
| d | = | doublet | br | = | broad |
| t | = | triplet | ppm | = | parts per million |
| dd | = | double doublet | | | |

| Compound No. (Table) | Proton NMR Data (δ) |
|---|---|
| 1(1) | 1.93(3H,s), 3.64(3H,s), 3.79(3H,s), 4.95(2H,s), 7.00(2H,d), 7.10(1H,t), 7.15-7.37(6H,m), 7.49-7.55(1H,m), 7.58(1H,s) ppm. |
| 2(1) | 1.98(3H, s), 3.65(3H,s), 3.79(3H,s), 4.94(2H,s), 7.12-7.24(3H,m), 7.29-7.41(5H,m), 7.46-7.53(1H,m), 7.58(1H,s) ppm. |
| 3(1) | 1.85(3H,s); 3.66(3H,s); 3.80(3H,s); 4.99(2H,s); 7.12-7.19(2H,m); 7.23-7.39(4H,m); 7.51(2H,t); 7.58(1H,s); 7.64(1H,d) ppm. |
| 4(1) | MAJOR ISOMER: 2.03(3H,s); 3.63(3H,s); 3.78(3H,s); 4.95(2H,s); 7.05(2H,d); 7.15-7.21(1H,m); 7.30-7.38(2H,m); 7.42-7.56(4H,m); 7.57(1H,s) ppm.<br>MINOR ISOMER: 2.03(3H,s); 3.61(3H,s); 3.74(3H,s); 5.12(2H,s); 7.05(2H,d); 7.15-7.21(1H,m); 7.30-7.38(2H,m); 7.42-7.56(4H,m); 7.58(1H,s) ppm. |
| 5(1) | 1.97(3H,s); 3.66(3H,s); 3.80(3H,s); 4.94(2H,s); 6.89(1H,dd); 6.99-7.02(1H,m); 7.06(1H,dd); 7.15-7.28(4H,m); 7.32-7.39(2H,m); 7.47-7.53(1H,m); 7.59(1H,s) ppm. |
| 8(1) | 1.76(3H,s); 2.19(3H,s); 3.64(3H,s); 3.78(3H,s); 4.96(2H,s); 6.88(1H,brs); 7.00-7.06(1H,m); 7.08-7.21(4H,m); 7.28-7.38(2H,m); 7.51-7.58(1H,m); 7.58(1H,s) ppm. |
| 9(1) | 1.93(3H,s); 2.31(3H,s); 3.65(3H,s); 3.79(3H,s); 4.94(2H,s); 6.78-6.84(2H,m); 6.91(1H,d); 7.10-7.20(3H,m); 7.30-7.38(2H,m); 7.49-7.55(1H,m); 7.58(1H,s) ppm. |
| 10(1) | 1.88(3H,s); 2.31(3H,s); 3.65(3H,s); 3.78(3H,s); 4.94(2H,s); 6.90(2H,d); 7.03-7.11(3H,m); 7.13-7.19(1H,m); 7.30-7.37(2H,m); 7.49-7.55(1H,m); 7.59(1H,s) ppm. |
| 16(1) | 1.80(3H,s); 3.64(3H,s); 3.68(3H,s); 3.69(3H,s); 4.92(2H,s); 6.81(2H,d); 6.94(2H,d); 7.00-7.10(1H,brs); 7.11-7.18(1H,m); 7.30-7.37(2H,m); 7.49-7.57(1H,m); 7.58(1H,s) ppm. |
| 18(1) | 1.98(3H,s); 3.65(3H,s); 3.79(3H,s); 4.94(2H,s); 6.65-6.85(3H,m); 7.10-7.25(2H,m); 7.26(1H,s); 7.30-7.40(2H,m); 7.45-7.55(1H,m); 7.57(1H,s) ppm. |

| Compound No. (Table) | Proton NMR Data (δ) |
|---|---|
| 19(1) | 1.85(3H,s); 3.65(3H,s); 3.78(3H,s); 4.93(2H,s); 6.90-7.00(4H,m); 7.08(1H,s); 7.13-7.25(1H,m); 7.30-7.40(2H,m); 7.45-7.55(1H,m); 7.58(1H,s) ppm. |
| 21(1) | 1.80(3H,s); 3.66(3H,s); 3.79(3H,s); 4.95(2H,s); 6.78-6.90(3H,m); 7.00-7.11(1H,m); 7.11-7.19(1H,m); 7.28-7.39(2H,m); 7.49-7.54(1H,m); 7.58(1H,s) ppm. |
| 22(1) | 1.97(3H,s); 3.67(3H,s); 3.80(3H,s); 4.96(2H,s); 6.70-6.90(2H,m); 6.98-7.10(1H,m); 7.12-7.25(2H,m); 7.27-7.40(2H,m); 7.45-7.56(1H,m); 7.58(1H,s) ppm. |
| 23(1) | 1.79(3H,s); 3.68(3H,s); 3.80(3H,s); 4.97(2H,s); 6.60(1H,s); 6.88-7.00(2H,m); 7.10-7.23(2H,m); 7.25-7.40(2H,m); 7.50-7.58(1H,m); 7.60(1H,s) ppm. |
| 25(1) | 2.03(3H,s); 3.66(3H,s); 3.79(3H,s); 4.94(2H,s); 6.40-6.60(3H,m); 7.10-7.25(1H,m); 7.27-7.40(3H,m); 7.41-7.55(1H,m); 7.57(1H,s) ppm. |
| 26(1) | 1.78(3H,s); 3.68(3H,s); 3.81(3H,s); 4.97(2H,s); 6.68(1H,s); 7.10-7.25(1H,m); 7.30-7.40(2H,m); 7.45-7.55(1H,m); 7.59(1H,s) ppm. |
| 32(1) | 1.95(3H,s); 3.65(3H,s); 3.79(3H,s); 4.93(2H,s); 6.85-7.00(1H,m); 7.05-7.28(5H,m); 7.30-7.40(2H,m); 7.45-7.55(1H,m); 7.58(1H,s) ppm. |
| 36(1) | MAJOR ISOMER: 2.16(3H,s); 3.65(3H,s); 3.80(3H,s); 4.97(2H,s); 7.07(2H,d); 7.15-7.21(1H,m); 7.31-7.41(2H,m); 7.44-7.50(1H,m); 7.58(1H,s); 7.77(1H,brs); 8.14(2H,d) ppm. |
| | MINOR ISOMER: 2.10(3H,s); 3.69(3H,s); 3.81(3H,s); 4.98(2H,s); 7.07(2H,d); 7.15-7.21(1H,m); 7.31-7.41(2H,m); 7.44-7.50(1H,m); 7.59(1H,s); 7.77(1H,brs); 8.14(2H,d) ppm. |
| 87(1) | 1.83(3H,s); 2.45(3H,s); 3.65(3H,s); 3.78(3H,s); 5.00(2H,s); 7.02-7.08(2H,m); 7.15-7.19(1H,m); 7.18(1H,brs); 7.30-7.41(2H,m); 7.56-7.62(2H,m); 7.60(1H,s) ppm. |
| 88(1) | 2.19(3H,s); 2.37(3H,s); 3.62(3H,s); 3.80(3H,s); 5.00(2H,s); 6.78-6.83(1H,m); 7.12-7.18(1H,m); 7.26-7.39(3H,m); 7.48-7.53(1H,m); 7.56(1H,s); 7.66(1H,brs); 8.09-8.11(1H,m) ppm. |

| Compound No. (Table) | Proton NMR Data (δ) |
|---|---|
| 98(1) | 0.9-1.1(4H,m); 2.8-2.9(1H,m); 2.36(3H,s); 3.67(3H,s); 3.82(3H,s); 4.96(2H,s); 6.70(1H,d); 7.1-7.5(4H,m); 7.59(1H,s); 8.1(1H,brs); 8.15(1H,d) ppm. |
| 99(1) | 2.27(3H,s); 2.43(3H,s); 3.69(3H,s); 3.82(3H,s); 3.87(3H,s); 4.97(2H,s); 6.10(1H,s); 7.1-7.5(4H,m); 7.60(1H,s); 8.09(1H,brs) ppm. |
| 100(1) | 2.37(3H,s); 3.68(3H,s); 3.81(3H,s); 4.97(2H,s); 7.1-7.5(4H,s); 7.58(1H,s); 8.30(1H,brs); 8.34(2H,s) ppm. |
| 101(1) | 2.31(6H,s); 2.42(3H,s); 3.68(3H,s); 3.82(3H,s); 4.97(2H,s); 6.54(1H,s); 7.1-7.5(4H,m); 7.60(1H,s); 8.11(1H,brs) ppm. |
| 32(2) | 1.86(3H,s); 3.14(3H,s); 3.69(3H,s); 3.83(3H,s); 4.93(2H,s); 6.90-7.00(1H,m); 7.10-7.20(3H,m); 7.21-7.40(3H,m); 7.50-7.58(1H,m); 7.59(1H,s) ppm. |
| 99(2) | 2.19(3H,s); 2.28(3H,s); 3.23(3H,s); 3.65(3H,s); 3.76(3H,s); 3.86(3H,s); 5.00(2H,s); 5.97(1H,s); 7.00-7.5(4H,m); 7.54(1H,s) ppm. |
| 100(2) | 2.14(3H,s); 3.20(3H,s); 3.65(3H,s); 3.79(3H,s); 4.99(2H,s); 7.1-7.4(4H,m); 7.54(1H,s); 8.30(2H,s) ppm. |
| 101(2)* | 2.18(3H,s); 2.31(6H,s); 3.25(3H,s); 3.65(3H,s); 3.78(3H,s); 5.00(2H,s); 6.40(1H,s); 7.1-7.5(4H,m); 7.55(1H,s) ppm. |
| 8(5) | 1.72(3H,s); 2.20(3H,s); 3.78(3H,s); 4.01(3H,s); 4.93(2H,s); 6.77(1H,brs); 7.00-7.20(5H,m); 7.34-7.47(2H,m); 7.50-7.55(1H,m) ppm. |

* A 93:7 mixture with Compound 101(1).

Compounds of formula (IV) (that is, compounds of formula (I) wherein $R^1$ is hydrogen) can be prepared by reacting a compound of formula (II) with a compound of formula (III) optionally in the presence of a base (such as triethylamine or pyridine) optionally in a convenient solvent (such as diethyl ether or tetrahydrofuran) at a temperature of 20-110°C.

Compounds of formula (I), wherein $R^1$ is as defined above but other than hydrogen, can be prepared by reacting a compound of formula (IV) with a compound of formula $R^1L$ (wherein $R^1$ is not hydrogen) in the presence of a base (such as sodium hydride, potassium carbonate or triethylamine) in a convenient solvent (such as diethyl ether or tetrahydrofuran) at a temperature of 20-110°C.

Examples of the L group are halogen (including fluorine, bromine and iodine but preferably chlorine) or $MeOSO_2$. The L group of a compound of formula (II) may also be methoxy.

Compounds of formula (II), wherein L is other than methoxy, can be prepared by reacting amides of formula (V) with a halogenating agent such as phosphorus trichloride, phosphorus pentachloride, phosphorus tribromide, phosphorus pentabromide, phosphorus oxychloride or phosphorus oxybromide optionally in a convenient solvent such as tetrahydrofuran.

A compound of formula (II), wherein L is methoxy, can be prepared by reacting an amine of formula $R^5NH_2$ with trimethylorthoacetate in the presence of a Lewis Acid (such as zinc (II) chloride, aluminium trichloride or boron trifluoride etherate), optionally in a suitable solvent.

Compounds of formula (IV) can exist as the tautomeric form (IVa) but they usually exist in the form (IV).

In another aspect the present invention provides a process for the preparation of a compound of formula (I).

The compounds are active fungicides and may be used to control one or more of the following pathogens: Pyricularia oryzae on rice and wheat and other Pyricularia spp. on other hosts; Puccinia recondita, Puccinia striiformis and other rusts on wheat, Puccinia hordei, Puccinia striiformis and other rusts on barley, and rusts on other hosts e.g. turf, rye, coffee, pears, apples, peanuts, sugar beet, vegetables and ornamental plants; Erysiphe graminis (powdery mildew) on barley, wheat, rye and turf and other powdery mildews on various hosts such as Sphaerotheca macularis on hops, Sphaerotheca fuliginea on cucurbits (e.g. cucumber), Podosphaera leucotricha on apple and Uncinula necator on vines; Cochliobolus spp., Helminthosporium spp., Drechslera spp. (Pyrenophora spp.), Rhynchosporium spp., Septoria spp. (including Mycosphaerella graminicola and Leptosphaeria nodorum), Pseudocercosporella herpotrichoides and Gaeumannomyces graminis on cereals (e.g. wheat, barley, rye), turf and other hosts; Cercospora arachidicola and Cercosporidium personatum on peanuts and other Cercospora species on other hosts, for example, sugar beet, bananas, soya beans and rice; Botrytis cinerea (grey mould) on tomatoes, strawberries, vegetables, vines and other hosts and other Botrytis spp. on other hosts; Alternaria spp. on vegetables (e.g. cucumber), oil-seed rape, apples, tomatoes, cereals (e.g. wheat) and other hosts; Venturia spp. (including Venturia inaequalis (scab)) on apples, pears, stone fruit, tree nuts and other hosts; Cladosporium spp. on a range of hosts including cereals (e.g. wheat); Monilinia spp. on stone fruit, tree nuts and other hosts; Didymella spp. on tomatoes, turf, wheat and other hosts; Phoma spp. on oil-seed rape, turf, rice, potatoes, wheat and other hosts; Aspergillus spp. and Aureobasidium spp. on wheat, lumber and other hosts; Ascochyta spp. on peas, wheat, barley and other hosts; Plasmopara viticola on vines; other downy mildews such as Bremia lactucae on lettuce, Peronospora spp. on soybeans, tobacco, onions and other hosts, Pseudoperonospora humuli on hops and Pseudoperonospora cubensis on cucurbits; Pythium spp. on turf and other hosts; Phytophthora infestans on potatoes and tomatoes and other Phytophthora spp. on vegetables, strawberries, avocado, pepper, ornamentals, tobacco, cocoa and other hosts; Thanatephorus cucumeris on rice and turf and other Rhizoctonia species on various hosts such as wheat and barley, vegetables, cotton and turf; Sclerotinia spp. on turf, peanuts, oil-seed rape and other hosts; Sclerotium spp. on turf, peanuts and other hosts; Colletotrichum spp. on a range of hosts including turf, coffee and vegetables; Laetisaria fuciformis on turf; Mycosphaerella spp. on banana, peanut, citrus, pecan, papaya and other hosts; Diaporthe spp. on citrus, soybean, melon, pear, lupin and other hosts; Elsinoe spp. on citrus, vines, olives, pecans, roses and other hosts; Pyrenopeziza spp. on oil-seed rape and other hosts; Oncobasidium theobromae on cocoa causing vascular streak dieback; Fusarium spp., Typhula spp., Microdochium nivale, Ustilago spp., Urocystis spp., Tilletia spp., and Claviceps purpurea on a variety of hosts but particularly wheat, barley, turf and maize; Ramularia spp. on sugar beet and other hosts; post-harvest diseases particularly of fruit (e.g. Pencillium digitatum and P. italicum and Trichoderma viride on oranges, Colletotrichum musae and Gloeosporium musarum on bananas and Botrytis cinerea on grapes); other pathogens on vines, notably Eutypa lata, Guignardia bidwellii, Phellinus igniarus, Phomopsis viticola, Pseudopezicula tracheiphila and Stereum hirsutum; other pathogens on lumber, notably Cephaloascus fragrans, Ceratocystis spp., Ophiostoma piceae, Penicillium spp., Trichoderma pseudokoningii, Trichoderma viride Trichoderma harzianum, Aspergillus niger, Lentographium lindbergi and Aureobasidium pullulans; and fungal vectors of viral diseases e.g. Polymyxa graminis on cereals as the vector of barley yellow mosaic virus (BYMV).

Some of the compositions show a broad range of activities against fungi in vitro.

Further, some of the compositions may be active as seed dressings against pathogens including Fusarium spp., Septoria spp., Tilletia spp., (e.g. bunt, a seed-borne disease of wheat), Ustilago spp. and Helminthosporium spp. on cereals, Rhizoctonia solani on cotton and Pyricularia oryzae on rice.

The compounds may move acropetally/locally in plant tissue. Moreover, the compounds may be volatile enough to be active in the vapour phase against fungi on the plant.

The invention therefore provides a method of combating fungi which comprises applying to a plant, to a seed of a plant or to the locus of the plant or seed a fungicidally effective amount of a compound as hereinbefore defined, or a composition containing the same.

The compounds may be used directly for agricultural purposes but are more conveniently formulated into compositions using a carrier or diluent. The invention thus provides fungicidal compositions comprising a compound as hereinbefore defined and an acceptable carrier or diluent therefor. It is preferred that all compositions, both solid and liquid formulations, comprise 0.0001 to 95%, more preferably 1 to 85%, for example 1 to 25% or 25 to 60%, of a compound as hereinbefore defined.

When applied the foliage of plants, the compounds of the invention are applied at rates of 0.1g to 10kg, preferably 1g to 8kg, more preferably 10g to 4kg, of active ingredient (invention compound) per hectare.

When used as seed dressings, the compounds of the invention are used at rates of 0.0001g (for example 0.001g

or 0.05g) to 10g, preferably 0.005g to 8g, more preferably 0.005g to 4g, of active ingredient (invention compound) per kilogram of seed.

The compounds can be applied in a number of ways. For example, they can be applied, formulated or unformulated, directly to the foliage of a plant, to seeds or to other medium in which plants are growing or are to be planted, or they can be sprayed on, dusted on or applied as a cream or paste formulation, or they can be applied as a vapour or as slow release granules.

Application can be to any part of the plant including the foliage, stems, branches or roots, or to soil surrounding the roots, or to the seed before it is planted, or to the soil generally, to paddy water or to hydroponic culture systems. The invention compounds may also be injected into plants or sprayed onto vegetation using electrodynamic spraying techniques or other low volume methods.

The term "plant" as used herein includes seedlings, bushes and trees. Furthermore, the fungicidal method of the invention includes preventative, protectant, prophylactic, systemic and eradicant treatments.

The compounds are preferably used for agricultural and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example, fillers such as kaolin, bentonite, kieselguhr, dolomite, calcium carbonate, talc, powdered magnesia, fuller's earth, gypsum, diatomaceous earth and china clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example, a mineral oil) for assisting the adhesion of the composition to the seed; alternatively the active ingredient can be formulated for seed dressing purposes using an organic solvent (for example, N-methylpyrrolidone, propylene glycol or N,N-dimethylformamide). The compositions may also be in the form of water dispersible powders or water dispersible granules comprising wetting or dispersing agents to facilitate the dispersion in liquids. The powders and granules may also contain fillers and suspending agents.

The compositions may also be in the form of soluble powders or granules, or in the form of solutions in polar solvents.

Soluble powders may be prepared by mixing the active ingredient with a water-soluble salt such as sodium bicarbonate, sodium carbonate, magnesium sulphate or a polysaccharide, and a wetting or dispersing agent to improve water dispersibility/solubility. The mixture may then be ground to a fine powder. Similar compositions may also be granulated to form water-soluble granules. Solutions may be prepared by dissolving the active ingredient in polar solvents such as ketones, alcohols and glycol ethers. These solutions may contain surface active agents to improve water dilution and prevent crystallisation in a spray tank.

Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes ketones such as cyclohexanone and methylcyclohexanone, chlorinated hydrocarbons such as chlorobenzene and trichlorethane, and alcohols such as benzyl alcohol, furfuryl alcohol, butanol and glycol ethers.

Aqueous suspension concentrates of largely insoluble solids may be prepared by ball or bead milling with a dispersing agent with a suspending agent included to stop the solid settling.

Compositions to be used as sprays may be in the form of aerosols wherein the formulation is held in a container under pressure of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds.

Alternatively, the compounds may be used in micro-encapsulated form. They may also be formulated in biodegradable polymeric formulations to obtain a slow, controlled release of the active substance.

By including suitable additives, for example additives for improving the uptake, distribution, adhesive power and resistance to rain on treated surfaces, the different compositions can be better adapted for various utilities. Other additives may be included to improve the biological efficacy of the various formulations. Such additives can be surface active materials to improve the wetting and retention on surfaces treated with the formulation and also the uptake and mobility of the active material, or additionally can include oil based spray additives, for example, certain mineral oil and natural plant oil (such as soya bean and rape seed oil) additives, or blends of them with other adjuvants.

The invention compounds can be used as mixtures with fertilisers (e.g. nitrogen-, potassium- or phosphorus-containing fertilizers). Compositions comprising only granules of fertilizer incorporating, for example coated with, a compound of formula (I) are preferred. Such granules suitably contain up to 25% by weight of the compound. The invention therefore also provides a fertilizer composition comprising a fertiliser and the compound of general formula (I) or a salt or metal complex thereof.

Water dispersible powders, emulsifiable concentrates and suspension concentrates will normally contain surfactants, e.g. a wetting agent, dispersing agent, emulsifying agent or suspending agent. These agents can be cationic,

anionic or non-ionic agents.

Suitable cationic agents are quaternary ammonium compounds, for example, cetyltrimethylammonium bromide. Suitable anionic agents are soaps, salts of aliphatic monoesters of sulphuric acid (for example, sodium lauryl sulphate), and salts of sulphonated aromatic compounds (for example, sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, alkyl glucosides, polysaccharides and the lecithins and the condensation products of the said partial esters with ethylene oxide. Suitable suspending agents are hydrophilic colloids (for example, polyvinylpyrrolidone and sodium carboxymethylcellulose), and swelling clays such as bentonite or attapulgite.

Compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 1-85%, for example 1-25% or 25-60%, by weight of the active ingredient. After dilution to form aqueous preparations, such preparations may contain varying amounts of the active ingredient depending upon the intended purpose, but an aqueous preparation containing 0.0001 to 10%, for example 0.005 to 10%, by weight of active ingredient may be used.

The compositions of this invention may contain other compounds having biological activity, e.g. compounds having similar or complementary fungicidal activity or which possess plant growth regulating, herbicidal or insecticidal activity.

An additional fungicidal compound may be present in the composition of the invention. By including another fungicide, the resulting composition can have a broader spectrum of activity or a greater level of intrinsic activity than the compound of general formula (I) alone. Further the other fungicide can have a synergistic effect on the fungicidal activity of the compound of general formula (I). Examples of fungicidal compounds which may be included in the composition of the invention are (RS)-1-aminopropylphosphonic acid, (RS)-4-(4-chlorophenyl)-2-phenyl-2-(1H-1,2,4-triazol-1-ylmethyl)butyronitrile, (Z)-N-but-2-enyloxymethyl-2-chloro-2',6'-diethylacetanilide, 1-(2-cyano-2-methoxyiminoacetyl)-3-ethyl urea, 4-(2,2-difluoro-1,3-benzodioxol-4-yl)pyrrole-3-carbonitrile, 4-bromo-2-cyano-N,N-dimethyl-6-trifluoromethylbenzimidazole-1-sulphonamide 5-ethyl-5,8-dihydro-8-oxo(1,3)-dioxol-(4,5-g)quinoline-7-carboxylic acid, α-[N-(3-chloro-2,6-xylyl)-2-methoxyacetamido]-g-butyrolactone, N-(2-methoxy-5-pyridyl)-cyclopropane carboxamide, alanycarb, aldimorph, ampropylfos, anilazine, azaconazole, BAS 490F, benalaxyl, benomyl, biloxazol, binapacryl, bitertanol, blasticidin S, bromuconazole, bupirimate, butenachlor, buthiobate, captafol, captan, carbendazim, carbendazim chlorhydrate, carboxin, chinomethionate, chlorbenzthiazone, chloroneb, chlorothalonil, chlorozolinate, clozylacon, copper containing compounds such as copper oxychloride, copper oxyquinolate, copper sulphate, copper tallate, and Bordeaux mixture, cycloheximide, cymoxanil, cyproconazole, cyprodinyl, cyprofuram, debacarb, di-2-pyridyl disulphide 1,1'-dioxide, dichlofluanid, dichlone, diclobutrazol, diclomezine, dicloran, didecyl dimethyl ammonium chloride, diethofencarb, difenoconazole, O,O-di-iso-propyl-S-benzyl thiophosphate, dimefluazole, dimetconazole, dimethomorph, dimethirimol, diniconazole, dinocap, dipyrithione, ditalimfos, dithianon, dodemorph, dodine, doguadine, edifenphos, epoxiconazole, etaconazole, ethirimol, ethoxyquin, ethyl (Z)-N-benzyl-N-([methyl(methylthioethylideneaminooxycarbonyl)amino]thio)-β-alaninate, etridiazole, fenaminosulph, fenapanil, fenarimol, fenbuconazole, fenfuram, fenpiclonil, fenpropidin, fenpropimorph, fentin acetate, fentin hydroxide, ferbam, ferimzone, fluazinam, fludioxonil, fluoroimide, fluquinconazole, fluzilazole, flutolanil, flutriafol, folpet, fuberidazole, furametpyr, furalaxyl, furconazole-cis, guazatine, hexaconazole, hydroxyisoxazole, hymexazole, ICIA5504, imazalil, imibenconazole, ipconazole, iprobenfos, iprodione, isopropanyl butyl carbamate, isoprothiolane, kasugamycin, mancozeb, maneb, mepanipyrim, mepronil, metalaxyl, metconazole, methfuroxam, metiram, metiram-zinc, metsulfovax, myclobutanil, NTN0301, neoasozin, nickel dimethyldithiocarbamate, nitrothal-isopropyl, nuarimol, ofurace, organomercury compounds, oxadixyl, oxolinic acid, oxycarboxin, pefurazoate, penconazole, pencycuron, phenazin oxide, phosetyl-Al, phosphorus acids, phthalide, polyoxin D, polyram, probenazole, prochloraz, procymidone, propamocarb, propamocarb hydrochloride, propiconazole, propineb, propionic acid, prothiocarb, pyracarbolid, pyrazophos, pyrifenox, pyrimethanil, pyroquilon, pyroxyfur, pyrrolnitrin, quaternary ammonium compounds, quinconazole, quinomethionate, quintozene, rabenazole, sodium pentachlorophenate streptomycin, sulphur, tebuconazole, techlofthalam, tecnazene, tetraconazole, thiabendazole, thicyofen, thifluzamide, 2-(thiocyanomethylthio)benzothiazole, thiophanate-methyl, thiram, timibenconazole, tolclofos-methyl, tolylfluanid, triacetate salt of 1,1'-iminodi(octamethylene)diguanidine, triadimefon, triadimenol, triazbutyl, triazoxide, tricyclazole, tridemorph, triforine, triflumizole, triticonazole, validamycin A, vapam, vinclozolin, XRD-563, zineb and ziram. The compounds of general formula (I) can be mixed with soil, peat or other rooting media for the protection of plants against seed-borne, soil-borne or foliar fungal diseases.

The compounds of formula (I) may also be used to combat and control infestations of insect pests and also other

invertebrate pests, for example, acarine pests. The insect and acarine pests which may be combated and controlled by the use of the invention compounds include those pests associated with agriculture (which term includes the growing of crops for food and fibre products), horticulture and animal husbandry, forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The insecticidal compositions may also comprise another pesticidal material, for example another insecticide or acaricide, or a fungicide, or may also comprise an insecticide synergist, such as for example dodecyl imidazole, safroxan, or piperonyl butoxide.

The insecticidal compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice.

Alternatively the insecticidal compositions may be in the form of baits wherein the active ingredient is mixed with a nutrient carrier for example sucrose, yeast, malt extract, cereal or cereal products and optionally an attractant such as a pheromone or pheromone analogue.

Alternatively the insecticidal compositions may be in the form of liquid preparations to be used as dips or sprays, which are generally aqueous dispersions or emulsions of the active ingredient in the presence of one or more known wetting agents, dispersing agents or emulsifying agents (surface active agents) of the type described above for the fungicidal compositions.

The insecticidal compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydrofurfuryl alcohol (THFA).

The insecticidal compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogeneous for a sufficient time to enable them to be applied by conventional spray equipment. The concentrates may contain 10-85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient (approximately equivalent to from 5-2000g/ha) is particularly useful.

In use the insecticidal compositions are applied to the pests, to the locus of the pests, to the habitat of the pests, to growing plants liable to infestation by the pests, or, where there is systemic uptake by plants, to the soil surrounding plants liable to infestation, by any of the known means of applying pesticidal compositions, for example, by dusting or spraying.

The compounds of the invention may be the sole active ingredient of the insecticidal composition or they may be admixed with one or more additional active ingredients such as insecticides, insecticide synergists, herbicides, fungicides or plant growth regulators where appropriate. Suitable additional active ingredients for inclusion in admixture with the compounds of the invention may be compounds which will broaden the spectrum of activity of the compounds of the invention or increase their persistence in the location of the pest. They may synergise the activity of the compound of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following:

a) Pyrethroids such as permethrin, esfenvalerate$_9$ deltamethrin, cyhalothrin in particular lambda-cyhalothrin, cypermethrin, alpha cypermethrin, bifenthrin, fenpropathrin, cyfluthrin, tefluthrin, fish safe pyrethroids for example ethofenprox, natural pyrethrin, tetramethrin, s-bioallethrin, fenfluthrin, prallethrin, or 5-benzyl-3-furylmethyl-(E)-(1R,3S)-2,2-dimethyl-3-(2-oxothiolan-3-ylidenemethyl) cyclopropane carboxylate;

b) Organophosphates such as profenofos, sulprofos, methyl parathion, azinphos-methyl, demeton-s-methyl, heptenophos, thiometon, fenamiphos, monocrotophos, triazophos, methamidophos, dimethoate, phosphamidon, malathion, chloropyrifos, phosalone, fensulfothion, fonofos, phorate, phoxim, pirimiphos-methyl, fenitrothion or diazinon;

c) Carbamates (including aryl carbamates) such as pirimicarb, cloethocarb, carbofuran, ethiofencarb, aldicarb, thiofurox, carbosulfan, bendiocarb, fenobucarb, propoxur or oxamyl;

d) Benzoyl ureas such as triflumuron, or chlorfluazuron;

e) Organic tin compounds such as cyhexatin, fenbutatin oxide, or azocyclotin;

f) Macrolides such as avermectins or milbemycins, for example such as abamectin, ivermectin, and milbemycin;

g) Hormones such as pheromones;

h) Organochlorine compounds such as benzene hexachloride, DDT, chlordane or dieldrin.

i) Amidines, such as chlordimeform or amitraz.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin can be employed. Alternatively insecticides specific for particular insect species/stages for example ovo-larvicides such as clofentezine, flubenzimine, hexythiazox and tetradifon, acaricides such as dicofol, propargite, bromopropylate, chlorobenzilate, or growth regulators such as hydramethylnon, cyromazine, methoprene$_9$ hydroprene, chlorfluazuron and diflubenzuron may also be included in the compositions.

Examples of suitable insecticide synergists for use in the compositions include piperonyl butoxide, sesamex, and dodecyl imidazole.

Suitable herbicides, fungicides and plant-growth regulators for inclusion in the compositions will depend upon the intended target and the effect required. An example of a rice selective herbicide which can be included is propanil, an example of a plant growth regulator for use in cotton is "Pix", and examples of fungicides for use in rice include blasticides such as blasticidin-S.

The ratio of the compound of the invention to the other active ingredient in the composition will depend upon a number of factors including type of target, effect required from the mixture etc.

However in general, the additional active ingredient of the composition will be applied at about the rate as it is usually employed, or at a slightly lower rate if synergism occurs.

The compounds of formula I and insecticidal compositions comprising them have shown themselves active against a variety of insect and other invertebrate pests. They are particularly useful in controlling sucking pests such as aphids and mites. Compounds of the present invention are also generally characterised by a relatively broad spectrum of activity which may include Lepidoptera and Coleoptera in addition to public health pests such as flies and cockroaches.

They may also be active against organophosphate, pyrethroid or cyclodiene (for example lindane or dieldrin) resistant strains of public and animal health pests. They may be effective in combating both susceptible and resistant strains of the pests in their adult and immature stages of growth, and may be applied to the infested host animal by topical, oral or parenteral administration.

The following Examples illustrate the invention.

## EXAMPLE 1

This Example illustrates the preparation of (E),(E)-methyl 2-[2-(anilino-acetoximinomethyl)phenyl]-3-methoxypropenoate (Compound No. 1 in Table 1).

### Stage 1

Phosphorus pentachloride (4.01g, 19.3mmol) was added to a solution of acetanilide (2.60g, 19.3mmol) in dry tetrahydrofuran (25ml) under a nitrogen atmosphere. The solution was stirred at room temperature for 15 minutes and heated at 60°C for 5 minutes. After cooling to room temperature the solvent and phosphorus oxychloride were removed under reduced pressure to give a crude imidoyl chloride which was used in the final stage without further purification.

### Stage 2

(E)-Methyl 2-[phthalimidooxymethylphenyl]-3-methoxy-propenoate (0.842g) (prepared as described in Example 4 of EP 0463488) was suspended in methanol (20ml) at room temperature, hydrazine hydrate (0.115g) was added and the resulting solution stirred for 3 hours. The white precipitate which formed was filtered off and the solvent removed to give a white semi-solid. This was diluted with diethyl ether, the white solid filtered off and the filtrate evaporated to give (E)-methyl 2-[2-aminooxymethylphenyl]-3-methoxypropenoate (93%) as a yellow oil which was used immediately in the next stage without further purification.

Stage 3

A solution of (E)-methyl 2-[2-aminooxymethylphenyl]-3-methoxy-propenoate from Stage 2 in dry tetrahydrofuran (5ml) was added dropwise to a solution of the imidoyl chloride from Stage 1 in dry tetrahydrofuran (5ml). After stirring at room temperature for 1 hour, triethylamine (2ml) was added and stirring continued for a further 8 hours . The white precipitate that formed was filtered off and the solvent removed from the filtrate to give a yellow gum. This gum was purified by hplc (silica eluted with diethyl ether:hexane 1:1) to give the title compound (0.053g, 7%) as a clear gum.

EXAMPLE 2

This Example illustrate the preparation of (E),(E)-methyl 2-[2-(3-trifluoromethylanilino-acetoximinomethyl)phenyl]-3-methoxypropenoate (Compound No. 2 in Table 1).

Stage 1

Phosphorus pentachloride (0.661g, 3.18mmol) was added to a solution of 3-trifluoromethylacetanilide (0.645g, 3.18mmol) in dry tetrahydrofuran (10ml) under a nitrogen atmosphere. The solution was stirred at room temperature for 15 minutes and heated at 60°C for 5 minutes. After cooling to room temperature the solvent and phosphorus oxychloride were removed under reduced pressure to give a crude imidoyl chloride which was used in the final stage without further purification.

Stage 2

(E)-Methyl 2-[phthalimidooxymethylphenyl]-3-methoxypropenoate (0.972g) (prepared as described in Example 4 of EP 0463488) was suspended in methanol (20ml) at room temperature, hydrazine hydrate (0.132g) was added and the resulting solution stirred for 3 hours. The white precipitate which formed was filtered off and the solvent removed to give a white semi-solid. This was diluted with diethyl ether, the white solid filtered off and the filtrate evaporated to give (E)-methyl 2-[2-aminooxymethyl-phenyl]-3-methoxypropenoate (93%) as a yellow oil which was used immediately in the next stage without further purification.

Stage 3

A solution of (E)-methyl-2-[2-aminooxymethylphenyl]-3-methoxy-propenoate from Stage 2 in dry tetrahydrofuran (5ml) was added dropwise to a solution of the imidoyl chloride from Stage 1 in dry tetrahydrofuran (5ml). After stirring at room temperature for 1 hour, triethylamine (2ml) was added, and the stirring continued for a further 8 hours. The white precipitate which formed was filtered off and the solvent removed from the filtrate to give a yellow gum. This gum was purified by hplc (silica eluted with dichloromethane:hexane: ethanol 25:50:2) to give the title compound (0.39g, 35%) as a yellow gum.

EXAMPLE 3

This Example illustrates the preparation of (E),(E)-methyl 2-[2-(2,4-difluoroanilinoacetoximinomethyl)phenyl]-3-methoxypropenoate (Compound No. 21 in Table 1).

Stage 1

A solution of 2,4-difluoroaniline (5.26g) and zinc (II) chloride (0.80g) in trimethylorthoacetate (4.86g) was heated to 70°C for two hours. This solution was poured directly onto a silica gel chromatography column (hexane:ether 6:1 as eluant) to give a crude imidate (3.19g, 43%) as a yellow-orange oil.

Stage 2

(E)-Methyl 2-[phthalimidooxymethylphenyl]-3-methoxypropenoate (1.05g) (prepared as described in Example 4 of EP0463488) was suspended in methanol (25ml) at room temperature. Hydrazine hydrate (0.143g) was added to the suspension and the resulting solution stirred for 2½ hours. The white precipitate which formed was filtered off and the solvent removed to give a white semi-solid. This was diluted with diethyl ether, the white solid filtered off and the filtrate evaporated to give (E)-methyl 2-[2-aminooxymethylphenyl]-3-methoxypropenoate (91%) as a yellow oil which was used immediately in the next stage without further purification.

Stage 3

A solution of (E)-methyl 2-[2-aminooxymethylphenyl]-3-methoxypropenoate from Stage 2 in methanol (10ml) was added dropwise to a solution of the imidate produced in Stage 1 (0.582g) and pyridine (3 drops) in methanol (10ml). The reaction mixture was stirred overnight at room temperature. The solvent was removed to give a gum which was purified by flash column chromatography, using silica gel and hexane : diethyl ether 3:2 and 1:1 as eluants to give the title compound (0.342g, 31%) as a clear gum.

EXAMPLE 4

The compounds were tested against a variety of foliar fungal diseases of plants. The technique employed was as follows.

The plants were grown in John Innes Potting Compost (No 1 or 2) in 4 cm diameter minipots. The test compounds were formulated either by bead milling with aqueous DISPERSOL T or as a solution in acetone or acetone/ethanol which was diluted to the required concentration immediately before use. The formulations (100 ppm active ingredient) were sprayed on to the foliage or applied to the roots of the plants in the soil. The sprays were applied to maximum retention and the root drenches to a final concentration equivalent to approximately 40 ppm a.i. in dry soil. TWEEN 20 was added to give a final concentration of 0.05% when the sprays were applied to cereals.

For most of the tests the compounds were applied to the soil (roots) or to the foliage (by spraying) one or two days before the plant was inoculated with the disease. Exceptions were the test on Erysiphe graminis in which the plants were inoculated 24 hours before treatment and the test on Puccinia recondita in which the plants were inoculated 48 hours before treatment. Foliar pathogens were applied by spray as zoosporangial suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease level present (i.e. leaf area covered by actively sporulating disease) on each of the treated plants was recorded using the following assessment scale:

| | |
|---|---|
| 0 = 0% disease present | 20 and 24 = 10.1-20% disease present |
| 1 = 0.1-1% disease present | 30 = 20.1-30% disease present |
| 3 = 1.1-3% disease present | 60 = 30.1-60% disease present |
| 5 = 3.1-5% disease present | 90 and 94 = 60.1-100% disease present |
| 10 = 5.1-10% disease present | |

Each assessment was then expressed as a percentage of the level of disease present on the untreated control plants. This calculated value is referred to as a POCO (Percentage of Control) value. An example of a typical calculation is as follows:

Disease level on untreated control = 90

Disese level on treated plant = 30

$$\text{POCO} = \frac{\text{disease level on treated plant}}{\text{disease level on untreated control}} \times 100 = \frac{30}{90} \times 100 = 33.3$$

This calculated POCO value is then rounded to the nearest of the values in the 9-point assessment scale shown above. In this particular example, the POCO value would be rounded to 30. If the calculated POCO falls exactly mid-way between two of the points, it is rounded to the lower of the two values.

The results are shown in Table II.

TABLE II

| Compound No (Table No) | Pr | Egt | Sn | Pv | Pil | Po | Tc | Vi |
|---|---|---|---|---|---|---|---|---|
| 1(1) | 0a | 90a | 90a | 10a | 90a | 0a | 90a | 0a |
| 2(1) | 0 | 0 | 0 | 0 | 20 | 0 | 20 | 0 |
| 3(1) | 0 | 0 | 0 | 0 | 10 | 0 | 0 | 0 |
| 4(1) | 0 | 0 | - | 0 | 0 | - | 0 | - |
| 8(1) | 0 | 0 | 0 | 0 | 30 | 0 | 30 | 0 |
| 9(1) | 0 | 0 | 0 | 0 | - | 20 | 0 | 0 |
| 10(1) | - | 0 | 0 | 0 | 0 | 0 | 20 | 0 |
| 16(1) | 10 | 0 | 30 | 0 | 5 | 0 | 30 | 0 |
| 21(1) | 0 | 0 | 5 | 0 | 0 | - | 0 | - |
| 87(1) | 0 | 5 | 0 | 0 | 5 | 0 | 0 | 0 |
| 88(1) | 0 | 0 | 5 | 0 | 0 | 20 | 90 | 0 |
| 98(1) | 1 | 0 | 5 | 0 | 30 | 0 | 5 | - |
| 99(1) | 90 | 90 | 90 | - | 90 | 90 | 90 | 30 |
| 100(1) | 90a | 20a | 90a | 90a | 90a | - | 90a | 90a |
| 101(1) | 90a | 30a | 90a | 90a | 90a | - | 90a | 90a |
| 99(2) | 90 | 90 | 90 | 0 | 60 | - | 90 | 90 |
| 100(2) | 90a | 90a | 90a | 0a | 30a | 60a | 90a | 10a |
| 101(2) | 5 | 20 | 90 | 3 | 10 | 0 | 30 | 0 |
| 8(5) | 90 | 5 | 90 | 0 | 30 | 5 | 30 | 0 |

Pr = Puccinia recondita
Egt = Erysiphe graminis tritici
Sn = Septoria nodorum
Po = Pyricularia oryzae
Tc = Thanatephorus cucumeris
Vi = Venturia inaequalis
Pv = Plasmopara viticola
Pil = Phytophthera infestans
a = 10ppm foliar application only
- = no result

EXAMPLE 5

The activity of the compounds of formula (I) was determined using a variety of pests. The pests were treated with a liquid composition containing 250 parts per million (ppm) by weight of the compound except in the case of Meloidogyne incognita, which was treated with a liquid composition containing 6.25 ppm by weight of the compound in an in vitro test. The compositions were made by dissolving the compound in acetone and ethanol (50:50) mixture and diluting the solutions with water containing 0.1% by weight of a wetting agent sold under the trade name "SYNPERONIC" NP8 (further diluting to 6.25ppm in the case of Meloidogyne incognita) until the liquid composition contained the required concentration of the compound. "SYNPERONIC" is a Registered Trade Mark.

The test procedure adopted with regard to each pest was basically the same and comprised supporting a number of the pests on a medium which was usually a substrate, a host plant or a foodstuff on which the pests feed, and treating either or both the medium and the pests with the compositions. The mortality of the pests was then assessed at periods usually varying from two to five days after the treatment with the exception of Musca domestica, for which an additional initial knockdown assessment was made.

The results of the tests are presented in Table III for each of the compounds. The results indicate a grading of mortality (or knockdown in the case of Musca domestica), designated as A, B or C wherein A indicates 80-100% mortality, B indicates 40-79% mortality and C indicates 0-39% mortality. The pest species is designated by a letter code.

Information regarding the pest species, the support medium or food, and the type and duration of the test is given in Table IV.

TABLE III

| Compound No (Table No) | Tu | Mp | Md initial knockdown/kill | Hv | Se | Db | Mi |
|---|---|---|---|---|---|---|---|
| 2(1) | B | A | A/C | C | C | C | C |
| 4(1) | C | A | C/C | C | C | C | C |
| 8(1) | A | B | C/- | C | C | C | C |
| 9(1) | B | C | C/C | C | C | C | C |
| 10(1) | C | A | C/B | C | C | C | C |
| 21(1) | A | B | B/A | C | C | B | C |
| 87(1) | B | C | C/C | C | C | C | C |
| - No result | | | | | | | |

TABLE IV

| CODE LETTERS (TABLE 18) | TEST SPECIES | SUPPORT MEDIUM/FOOD | TYPE OF TEST | DURATION (DAYS) |
|---|---|---|---|---|
| Tu | Tetranychus urticae (spider mite) | French bean leaf | Contact | 3 |
| Mp | Myzus persicae (aphid) | Chinese Cabbage leaf | Contact | 3 |
| Md | Musca domestica (housefly - adult) | Cotton wool/sugar | Contact<br>Knockdown | 2<br>15 min |
| Hv | Heliothis virescens (tobacco budworm) | Soya leaf | Residual | 5 |
| Se | Spodoptera exigua (lesser armyworm - larva) | Cotton leaf | Residual | 5 |
| Db | Diabrotica balteata (cucumber beetle - larva) | Filter paper/maize seed | Residual | 2 |
| Mi | Meloidogyne incognita (rootknot nematode - larva) | in vitro | Contact | 2 |

"Contact" test indicates that both pests and medium were treated, "Residual" indicates that the medium was treated before infestation with the pests and "in vitro" indicates that the pest was suspended in an aqueous medium containing the treatment.

CHEMICAL FORMULAE (in description)

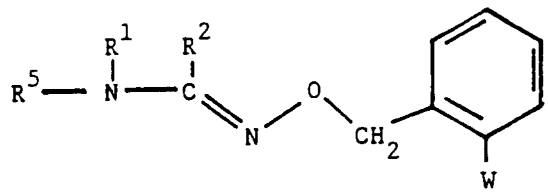

(I)

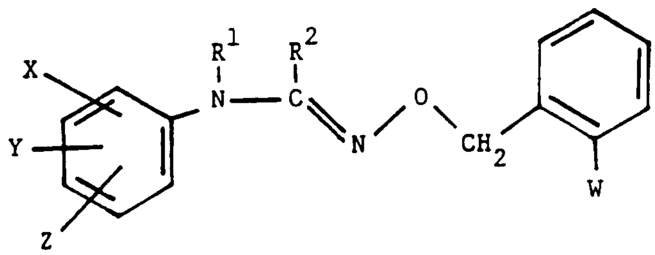

(Ia)

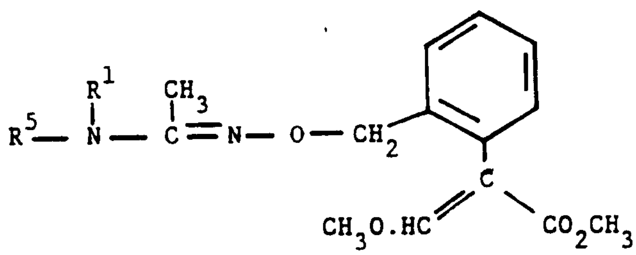

(Ib)

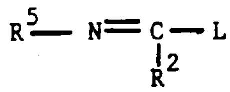

(II)

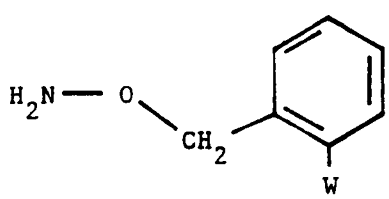

(III)

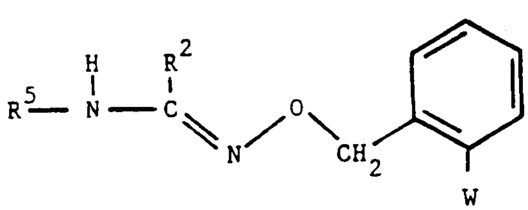

(IV)

CHEMICAL FORMULAE (in description)

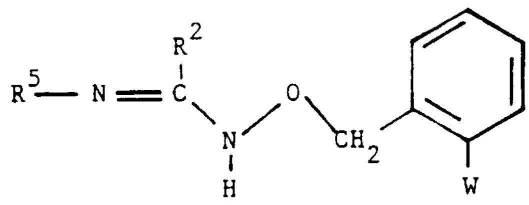

(IVa)

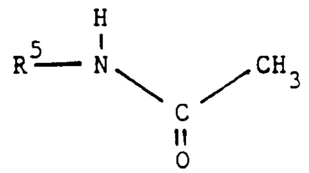

(V)

## Claims

1. A compound having the formula (I):

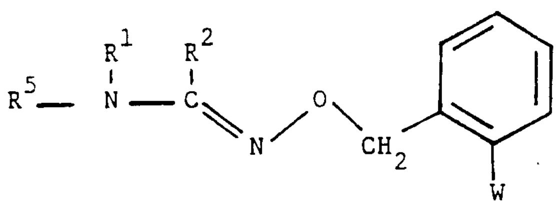

(I)

wherein $R^1$ is hydrogen, $C_{1-4}$ alkyl, $C_{1-4}$ haloalkyl, $C_{2-4}$ alkenyl, $C_{2-4}$ alkynyl, $C_{3-6}$ cycloalkyl, aryl or aryl($C_{1-4}$)alkyl; $R^2$ is hydrogen, $C_{1-4}$ alkyl, $C_{1-4}$ haloalkyl or $C_{3-6}$ cycloalkyl; W is $CH_3O.CH{=}CCO_2CH_3$, $CH_3O.N{=}CCONR^3R^4$, $CH_3O.CH{=}CCONR^3R^4$ or $CH_3O.N{=}CCO_2CH_3$ and stereoisomers thereof; and $R^5$ is a phenyl or heterocyclyl moiety optionally substituted by halogen, hydroxy, mercapto, $C_{1-4}$ alkyl, $C_{2-4}$ alkenyl, $C_{2-4}$ alkynyl, $C_{1-4}$ alkoxy, $C_{2-4}$ alkenyloxy, $C_{2-4}$ alkynyloxy, halo($C_{1-4}$)alkyl, halo($C_{1-4}$)alkoxy, $C_{1-4}$ alkylthio, hydroxy($C_{1-4}$)alkyl, $C_{1-4}$ alkoxy($C_{1-4}$)alkyl, $C_{3-6}$ cycloalkyl, $C_{3-6}$ cycloalkyl($C_{1-4}$)alkyl, optionally substituted methylenedioxy, optionally substituted aryl, optionally substituted heteroaryl, optionally substituted heterocyclyl, optionally substituted aryloxy, optionally substituted heteroaryloxy, optionally substituted aryl($C_{1-4}$)alkyl in which the alkyl moiety is optionally substituted with hydroxy, optionally substituted heteroaryl($C_{1-4}$)alkyl, optionally substituted aryl($C_{2-4}$)alkenyl, optionally substituted heteroaryl($C_{2-4}$)alkenyl, optionally substituted aryl($C_{1-4}$)alkoxy, optionally substituted heteroaryl($C_{1-4}$)alkoxy, optionally substituted aryloxy($C_{1-4}$)alkyl, optionally substituted heteroaryloxy($C_{1-4}$)alkyl, acyloxy, cyano, thiocyanato, nitro, -NR'R'', -NHCOR', -NHCONR'R'', -CONR'R'', -COOR', $-OSO_2R'$, $-SO_2R'$, -COR', -CR'=NR'' or -N=CR'R'' in which R' and R'' are independently hydrogen, $C_{1-4}$ alkyl, $C_{1-4}$ alkoxy, $C_{1-4}$ alkylthio, $C_{3-6}$ cycloalkyl, $C_{3-6}$ cycloalkyl($C_{1-4}$)alkyl, phenyl or benzyl, the phenyl and benzyl groups being optionally substituted with halogen, $C_{1-4}$ alkyl or $C_{1-4}$ alkoxy; when R' and R'' are in CONR'R'' they can together form a 5- or 6-membered heterocyclic ring; or two substituents, when they are in adjacent positions on the aryl or heteroaryl ring can join to form a fused alphatic ring; and $R^3$ and $R^4$ are independently hydrogen or $C_{1-4}$ alkyl.

2. A compound as claimed in claim 1 having the general formula (Ia):

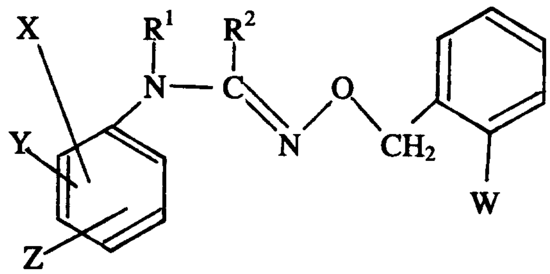

(Ia)

wherein $R^1$ is hydrogen, $C_{1-4}$ alkyl, $C_{1-4}$ haloalkyl, $C_{2-4}$ alkenyl, $C_{2-4}$ alkynyl, $C_{3-6}$ cycloalkyl, aryl or aryl($C_{1-4}$)alkyl; $R^2$ is hydrogen, $C_{1-4}$ alkyl, $C_{1-4}$ haloalkyl or $C_{3-6}$ cycloalkyl; W is $CH_3O.CH=CCO_2CH_3$, $CH_3O.N=CCONR^3R^4$, $CH_3O.CH=CCONR^3R^4$ or $CH_3O.N=CCO_2CH_3$ and stereoisomers thereof; X, Y and Z are independently hydrogen, halogen, hydroxy, mercapto, $C_{1-4}$ alkyl, $C_{2-4}$ alkenyl, $C_{2-4}$ alkynyl, $C_{1-4}$ alkoxy, $C_{2-4}$ alkenyloxy, $C_{2-4}$ alkynyloxy, halo($C_{1-4}$)alkyl, halo($C_{1-4}$)alkoxy, $C_{1-4}$ alkylthio, hydroxy($C_{1-4}$)alkyl, $C_{1-4}$ alkoxy($C_{1-4}$)alkyl, $C_{3-6}$ cycloalkyl, $C_{3-6}$ cycloalkyl($C_{1-4}$)alkyl, optionally substituted methylenedioxy, optionally substituted aryl, optionally substituted heteroaryl, optionally substituted heterocyclyl, optionally substituted aryloxy, optionally substituted heteroaryloxy, optionally substituted aryl($C_{1-4}$)alkyl in which the alkyl moiety is optionally substituted with hydroxy, optionally substituted heteroaryl($C_{1-4}$)alkyl, optionally substituted aryl($C_{2-4}$)alkenyl, optionally substituted heteroaryl($C_{2-4}$)alkenyl, optionally substituted aryl($C_{1-4}$)alkoxy, optionally substituted heteroaryl($C_{1-4}$)alkoxy, optionally substituted aryloxy($C_{1-4}$)alkyl, optionally substituted heteroaryloxy($C_{1-4}$)alkyl, acyloxy, cyano, thiocyanato, nitro, -NR'R", -NHCOR', -NHCONR'R", -CONR'R", -COOR', $-OSO_2R'$, $-SO_2R'$, -COR', -CR'=NR" or -N=CR'R" in which R' and R" are independently hydrogen, $C_{1-4}$ alkyl, $C_{1-4}$ alkoxy, $C_{1-4}$ alkylthio, $C_{3-6}$ cycloalkyl, $C_{3-6}$ cycloalkyl($C_{1-4}$)alkyl, phenyl or benzyl, the phenyl and benzyl groups being optionally substituted with halogen, $C_{1-4}$ alkyl or $C_{1-4}$ alkoxy; when R' and R" are in CONR'R" they can together form a 5- or 6-membered heterocyclic ring; or two substituents, when they are in adjacent positions on the aryl or heteroaryl ring can join to form a fused alphatic ring; and $R^3$ and $R^4$ are independently hydrogen or $C_{1-4}$ alkyl.

3. A compound as claimed in claim 1 or 2 wherein W is $CH_3O.CH=CCO_2CH_3$.

4. A compound as claimed in claim 1, 2 or 3 wherein $R^1$ is hydrogen or $C_{1-4}$ alkyl.

5. A compound as claimed in claim 1, 2, 3 or 4 wherein $R^2$ is methyl.

6. A compound as claimed in claim 1, 3, 4 or 5 having the formula (Ib):

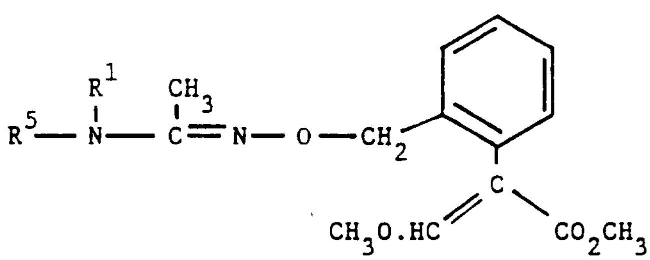

(Ib)

wherein $R^1$ is hydrogen or $C_{1-4}$ alkyl; $R^5$ is phenyl optionally substituted with halogen, $C_{1-4}$ alkyl, $C_{2-4}$ alkenyl, $C_{2-4}$ alkynyl, $C_{1-4}$ alkoxy, $C_{2-4}$ alkenyloxy, $C_{2-4}$ alkynyloxy, halo($C_{1-4}$)alkyl, halo($C_{1-4}$)alkoxy, $C_{1-4}$ alkoxy($C_{1-4}$)alkyl, acyloxy, cyano, thiocyanato, nitro, -NR'R", -NHCOR', -CONR'R", COOR' or COR' in which R' and R" are independently hydrogen, $C_{1-4}$ alkyl or $C_{1-4}$ alkoxy.

7. A compound as claimed in claim 6 wherein $R^1$ is hydrogen and $R^5$ is phenyl optionally substituted with halogen, cyano, nitro, halo($C_{1-4}$)alkyl or -COOR' in which R' is $C_{1-4}$ alkyl.

8. A process for preparing a compound as claimed in claim 1, the process comprising reacting a compound of formula (II):

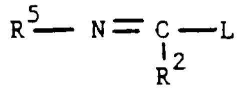

(II)

with a compound of formula (III):

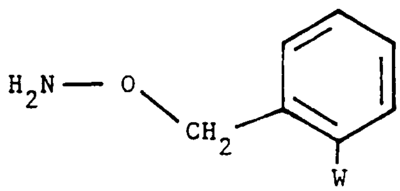

(III)

and, optionally, reacting the product formed with a compound of formula $R^1L$ (wherein $R^1$ is other than hydrogen).

9. A fungicidal composition comprising a fungicidally effective amount of a compound according to claim 1 and a fungicidally acceptable carrier or diluent therefor.

10. A method of combating fungi which comprises applying to plants, to the seeds of plants or to the locus of the plants or seeds, a compound according to claim 1 or a composition according to claim 9.

11. An insecticidal composition comprising an insecticidally or acaricidally effective amount of a compound according claim 1 and an insecticidally or acaricidally acceptable carrier or diluent therefor.

12. A method of combating insect or acarine pests which comprises applying to the locus of the pest, an insecticidally or acaricidally effective amount of a compound according to claim 1 or a composition according to claim 11.

**Patentansprüche**

1. Verbindung der Formel (I):

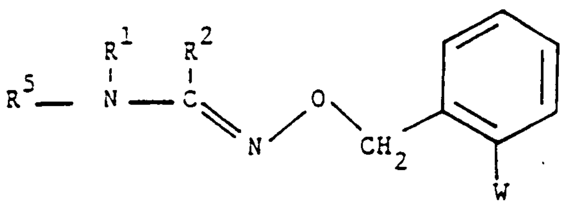

(I)

in der $R^1$ ein Wasserstoffatom, ein $C_{1-4}$-Alkyl-, $C_{1-4}$-Halogenalkyl-, $C_{2-4}$-Alkenyl-, $C_{2-4}$-Alkinyl-, $C_{3-6}$-Cydoalkyl-, Aryl- oder Aryl($C_{1-4}$)alkylrest ist; $R^2$ ein Wasserstoffatom, ein $C_{1-4}$-Alkyl-, $C_{1-4}$-Halogenalkyl- oder $C_{3-6}$-Cycloalkylrest ist; W ein Rest $CH_3O.CH{=}CCO_2CH_3$, $CH_3O.N{=}CCONR^3R^4$, $CH_3O.CH{=}CCONR^3R^4$ oder $CH_3O.N{=}CCO_2CH_3$ ist und Stereoisomere davon; und $R^5$ eine Phenyl- oder heterocyclische Einheit ist, gegebenenfalls substituiert mit Halogenatomen, Hydroxyl-, Mercaptogruppen, $C_{1-4}$-Alkyl-, $C_{2-4}$-Alkenyl-, $C_{2-4}$-Alkinyl-, $C_{1-4}$-Alkoxy-, $C_{2-4}$-Alkenyloxy-, $C_{2-4}$-Alkinyloxy-, Halogen($C_{1-4}$)alkyl-, Halogen($C_{1-4}$)-alkoxy-, $C_{1-4}$-Alkylthio-, Hydroxy($C_{1-4}$)alkyl-, $C_{1-4}$-Alkoxy($C_{1-4}$)alkyl-, $C_{3-6}$-Cycloalkyl-, $C_{3-6}$-Cycloalkyl($C_{1-4}$)alkylresten, gegebenenfalls substituierten Methylendioxyresten, gegebenenfalls substituierten Arylresten, gegebenenfalls substituierten Heteroarylresten, gegebenenfalls substituierten Heterocyclylresten, gegebenenfalls substituierten Aryloxyresten, gegebenenfalls substituierten Heteroaryloxyresten, gegebenenfalls substituierten Aryl($C_{1-4}$)alkylresten, wobei die Alkyleinheit gegebenenfalls mit Hydroxylgruppen substituiert ist, gegebenenfalls substituierten Heteroaryl($C_{1-4}$)alkylresten, gegebenenfalls substituierten Aryl($C_{2-4}$)alkenylresten, gegebenenfalls substituierten Heteroaryl($C_{2-}$

$_{4}$)alkenylresten, gegebenenfalls substituierten Aryl($C_{1-4}$)alkoxyresten, gegebenenfalls substituierten Heteroaryl($C_{1-4}$)alkoxyresten, gegebenenfalls substituierten Aryloxy($C_{1-4}$)alkylresten, gegebenenfalls substituierten Heteroaryloxy($C_{1-4}$)alkylresten, Acyloxyresten, Cyano-, Thiocyanato, Nitrogruppen, Resten - NR'R", -NHCOR', -NHCONR'R", -CONR'R", -COOR', -$OSO_2$R', -$SO_2$R', -COR', -CR'=NR" oder -N=CR'R", wobei R' und R" unabhängigvoneinander Wasserstoffatome, $C_{1-4}$-Alkyl-, $C_{1-4}$-Alkoxy-, $C_{1-4}$-Alkylthio-, $C_{3-6}$-Cycloalkyl-, $C_{3-6}$-Cycloalkyl($C_{1-4}$)alkylreste, Phenyl- oder Benzylgruppen sind, wobei die Phenyl- und Benzylgruppen gegebenenfalls mit Halogenatomen, $C_{1-4}$-Alkyl- oder $C_{1-4}$-Alkoxyresten substituiert sind; wenn R' and R" in dem Rest CONR'R" vorkommen, sie zusammen einen 5- oder 6-gliedrigen heterocyclischen Ring bilden können; oder zwei Substituenten, wenn sie sich in benachbarten Positionen an dem Aryl- oder Heteroarylring befinden, einen kondensierten aliphatischen Ring bilden können; und $R^3$ und $R^4$ unabhängig voneinander Wasserstoffatome oder $C_{1-4}$-Alkylreste sind.

2. Verbindung nach Anspruch 1 der allgemeinen Formel ( Ia ):

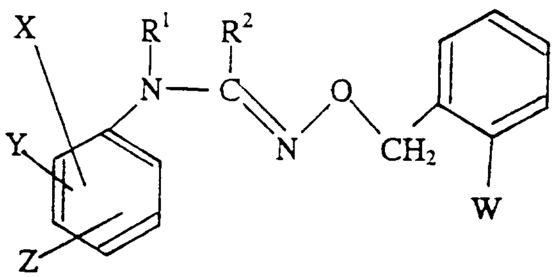

(Ia)

in der $R^1$ ein Wasserstoffatom, ein $C_{1-4}$-Alkyl-, $C_{1-4}$-Halogenalkyl-, $C_{2-4}$-Alkenyl-, $C_{2-4}$-Alkinyl-, $C_{3-6}$-Cycloalkyl-, Aryl- oder Aryl($C_{1-4}$)alkylrest ist; $R^2$ ein Wasserstoffatom, ein $C_{1-4}$-Alkyl-, $C_{1-4}$-Halogenalkyl oder $C_{3-6}$-Cycloalkylrest ist; W ein Rest $CH_3O.CH=CCO_2CH_3$, $CH_3O.N=CCONR^3R^4$, $CH_3O.CH=CCONR^3R^4$ oder $CH_3O.N=CCO_2CH_3$ ist und Stereoisomere davon; X, Y und Z bedeuten unabhängig voneinander Wasserstoff-, Halogenatome, Hydroxyl-, Mercaptogruppen, $C_{1-4}$-Alkyl-, $C_{2-4}$-Alkenyl-, $C_{2-4}$-Alkinyl-, $C_{1-4}$-Alkoxy-, $C_{2-4}$-Alkenyloxy-, $C_{2-4}$-Alkinyloxy-, Halogen($C_{1-4}$)alkyl-, Halogen($C_{1-4}$)-alkoxy-, $C_{1-4}$-Alkylthio-, Hydroxy($C_{1-4}$)alkyl-, $C_{1-4}$-Alkoxy($C_{1-4}$)alkyl-, $C_{3-6}$-Cycloalkyl-, $C_{3-6}$-Cycloalkyl($C_{1-4}$)alkylreste, gegebenenfalls substituierte Methylendioxyreste, gegebenenfalls substituierte Arylreste, gegebenenfalls substituierte Heteroarylreste, gegebenenfalls substituierte Heterocyclylreste, gegebenenfalls substituierte Aryloxyreste, gegebenenfalls substituierte Heteroaryloxyreste, gegebenenfalls substituierte Aryl($C_{1-4}$)alkylreste, wobei die Alkyleinheit gegebenenfalls mit Hydroxylgruppen substituiert ist, gegebenenfalls substituierte Heteroaryl($C_{1-4}$)alkylreste, gegebenenfalls substituierte Aryl($C_{2-4}$)alkenylreste, gegebenenfalls substituierte Heterorayl($C_{2-4}$)alkenylreste, gegebenenfalls substituierte Aryl($C_{1-4}$)alkoxyreste, gegebenenfalls substituierte Heteroaryl($C_{1-4}$)alkoxyreste, gegebenenfalls substituierte Aryloxy($C_{1-4}$)alkylreste, gegebenenfalls substituierte Heteroaryloxy($C_{1-4}$)alkylreste, Acyloxyreste, Cyano-, Thiocyanato-, Nitrogruppen, Reste -NR'R", -NHCOR', -NHCONR'R", -CONR'R", -COOR', -$OSO_2$R', -$SO_2$R', -COR', -CR'=NR" oder -N=CR'R", in denen R' und R" unabhängig voneinander Wasserstoffatome, $C_{1-4}$-Alkyl-, $C_{1-4}$-Alkoxy-, $C_{1-4}$-Alkylthio-, $C_{3-6}$-Cycloalkyl-, $C_{3-6}$-Cycloalkyl($C_{1-4}$)alkylreste, Phenyl- oder Benzylgruppen sind, wobei die Phenyl- und Benzylgruppen gegebenenfalls mit Halogenatomen, $C_{1-4}$-Alkyl- oder $C_{1-4}$-Alkoxyresten substituiert sind; wenn R' und R" in dem Rest CONR'R" vorkommen, sie zusammen einen 5- oder 6-gliedrigen heterocyclischen Ring bilden können; oder zwei Substituenten, wenn sie sich in benachbarten Positionen an dem Aryl- oder Heteroarylring befinden, einen kondensierten aliphatischen Ring bilden können; und $R^3$ und $R^4$ unabhängig voneinander Wasserstoffatome oder $C_{1-4}$-Alkylreste sind.

3. Verbindung nach Anspruch 1 oder 2, in der W eine Gruppe $CH_3O.CH=CCO_2CH_3$ ist.

4. Verbindung nach Anspruch 1, 2 oder 3, in der $R^1$ ein Wasserstoffatom oder ein $C_{1-4}$-Alkylrest ist.

5. Verbindung nach Anspruch 1, 2, 3 oder 4, in der $R^2$ eine Methylgruppe ist.

6. Verbindung nach Anspruch 1, 3, 4 oder 5 der Formel ( Ib ):

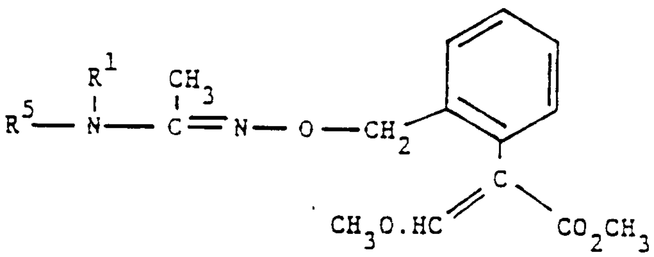

(Ib)

in der $R^1$ ein Wasserstoffatom oder ein $C_{1-4}$-Alkylrest ist; $R^5$ ein Phenylrest ist, der gegebenenfalls mit Halogenatomen, $C_{1-4}$-Alkyl-, $C_{2-4}$-Alkenyl-, $C_{2-4}$-Alkinyl-, $C_{1-4}$-Alkoxy-, $C_{2-4}$-Alkenyloxy-, $C_{2-4}$-Alkinyloxy-, Halogen($C_{1-4}$)alkyl-, Halogen($C_{1-4}$)-alkoxy-, $C_{1-4}$-Alkoxy($C_{1-4}$)alkyl-, Acyloxyresten, Cyano-, Thiocyanato-, Nitrogruppen, Resten -NR'R", -NHCOR', -CONR'R", -COOR' oder -COR' substituiert ist, wobei R' und R" unabhängig voneinander Wasserstoffatome, $C_{1-4}$-Alkyl- oder $C_{1-4}$-Alkoxyreste sind.

**7.** Verbindung nach Anspruch 6, in der $R^1$ ein Wasserstoffatom ist und $R^5$ ein Phenylrest ist, der gegebenenfalls mit Halogenatomen, Cyano-, Nitrogruppen, Resten Halogen($C_{1-4}$)alkyl oder -COOR' substituiert ist, wobei R' ein $C_{1-4}$-Alkylrest ist.

**8.** Verfahren zur Herstellung einer Verbindung nach Anspruch 1, umfassend das Umsetzen einer Verbindung der Formel ( II ):

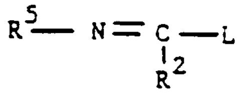

(II)

mit einer Verbindung der Formel ( III ):

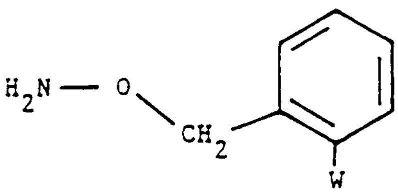

(III)

und gegebenenfalls Umsetzen des entstadenen Produktes mit einer Verbindung der Formel $R^1L$ ( in der $R^1$ kein Wasserstoffatom ist ).

**9.** Fungizid, umfassend eine fungizid wirksame Menge einer Verbindung nach Anspruch 1 und einen fungizid verträglichen Träger oder Verdünnungsmittel dafür.

**10.** Verfahren zur Bekämpfung von Pilzen, umfassend die Anwendung einer Verbindung nach Anspruch 1 oder einer Zusammensetzung nach Anspruch 9 an Pflanzen, dem Saatgut oder dem Standort der Pflanzen oder des Saatgutes.

**11.** Insektizid, umfassend eine insektizid oder akarizid wirksame Menge einer Verbindung nach Anspruch 1 und einen insektizid oder akarizid verträglichen Träger oder Verdünnungsmittel dafür.

**12.** Verfahren zur Bekämpfung von Schadinsekten oder Schadmilben, umfassend die Anwendung einer insektizid oder akarizid wirksamen Menge einer Verbindung nach Anspruch 1 oder einer Zusammensetzung nach Anspruch 11 am Ort des Schädlingsbefalls.

## Revendications

1. Composé ayant la formule (I) :

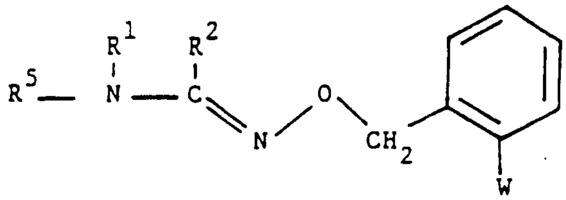

(I)

dans laquelle $R^1$ est l'hydrogène, un groupe alkyle en $C_1$-$C_4$, haloalkyle en $C_1$-$C_4$, alcényle en $C_2$-$C_4$, alcynyle en $C_2$-$C_4$, cycloalkyle en $C_3$-$C_6$, aryle ou alkyl (en $C_1$-$C_4$) aryle ; $R^2$ est l'hydrogène ou un groupe alkyle en $C_1$-$C_4$, haloalkyle en $C_1$-$C_4$ ou cycloalkyle en $C_3$-$C_6$ ; W est $CH_3O$. $CH=CCO_2CH_3$, $CH_3O$. $N=CCONR^3R^4$, $CH_3O$. $CH=CCO\ NR^3R^4$ ou $CH_3O$. $N=CCO_2\ CH_3$ et leurs stéréoisomères ; et $R^5$ est un fragment phényle ou hétérocyclyle éventuellement substitué par un halogène, un groupe hydroxy, mercapto, alkyle en $C_1$-$C_4$, alcényle en $C_2$-$C_4$, alcynyle en $C_2$-$C_4$, alcoxy en $C_1$-$C_4$, alcényloxy en $C_2$-$C_4$, alcynyloxy en $C_2$-$C_4$, haloalkyle en $C_1$-$C_4$, haloalcoxy en $C_1$-$C_4$, thioalkyle en $C_1$-$C_4$, hydroxyalkyle en $C_1$-$C_4$, alcoxy en $C_1$-$C_4$ alkyle en $C_1$-$C_4$, cycloalkyle en $C_3$-$C_6$, cycloalkyl en $C_3$-$C_6$ alkyle en $C_1$-$C_4$, méthylènedioxy éventuellement substitué, aryle éventuellement substitué, hétéroaryle éventuellement substitué, hétérocyclyle éventuellement substitué, aryloxy éventuellement substitué, hetéroaryloxy éventuellement substitué, arylalkyle en $C_1$-$C_4$ éventuellement substitué, dans lequel le segment alkyle est éventuellement substitué par un hydroxy, hétéroarylakyle en $C_1$- $C_4$ éventuellement substitué, arylalcényle en $C_2$-$C_4$ éventuellement substitué, hétéroarylalcényle en $C_2$-$C_4$ éventuellement substitué, arylalcoxy en $C_1$- $C_4$ éventuellement substitué, hétéroarylalcoxy en $C_1$- $C_4$ éventuellement substitué, aryloxy- alkyle en $C_1$- $C_4$ éventuellement substitué, hétéroaryloxyalkyle en $C_1$- $C_4$ éventuellement substitué, acyloxy, cyano, thiocyano, nitro, -NR'R", -NHCOR', -NHCONR'R", -CONR'R", -COOR', $-OSO_2R'$, $-SO_2R'$, -COR', -CR'-NR" ou -N=CR'R" dans lesquels R' et R" sont indépendamment l'hydrogène, un groupe alkyle en $C_1$-$C_4$, alcoxy en $C_1$-$C_4$, thioalkyle en $C_1$-$C_4$, cycloalkyle en $C_3$-$C_6$, cycloalkyl en $C_3$-$C_6$ alkyle en $C_1$-$C_4$, phényle ou benzyle, les groupes phényle et benzyle étant éventuellement substitués par un halogène, un groupe alkyle en $C_1$-$C_4$ ou alcoxy en $C_1$-$C_4$ ; quand R' et R" sont dans CONR'R" ils peuvent former ensemble un noyau hétérocyclique à 5 ou 6 atomes ; ou deux substituants, quand ils sont en positions adjacentes sur le noyau aryle ou hétéroaryle pouvant se joindre pour former un noyau aliphatique fusionné (en particulier pour former un noyau aliphatique carboné à 6 atomes) ; et $R^3$ et $R^4$ sont indépendamment l'hydrogène ou un groupe alkyle en $C_1$-$C_4$.

2. Composé selon la revendication 1, ayant la formule (Ia) :

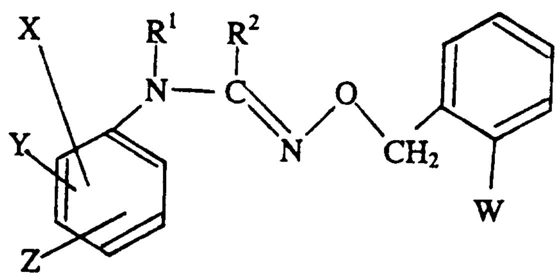

(Ia)

dans laquelle $R^1$ est l'hydrogène, un groupe alkyle en $C_1$-$C_4$, haloalkyle en $C_1$-$C_4$, alcényle en $C_2$-$C_4$, alcynyle en $C_2$-$C_4$, cycloalkyle en $C_3$-$C_6$, aryle ou arylalkyle en $C_1$-$C_4$; $R^2$ est l'hydrogène ou un groupe alkyle en $C_1$-$C_4$, haloalkyle en $C_1$-$C_4$ ou cycloalkyle en $C_3$-$C_6$ ; W est $CH_3O$. $CH=CCO_2CH_3$, $CH_3O$. $N=CCONR^3R^4$, $CH_3O$. $CH=CCO\ NR^3R^4$ ou $CH_3O$. $N=CCO_2\ CH_3$ et leurs stéréoisomères ; X, Y et Z sont indépendamment l'hydrogène, un groupe halogène, hydroxy, mercapto, alkyle en $C_1$-$C_4$, alcényle en $C_2$-$C_4$, alcynyle en $C_2$-$C_4$, alcoxy $C_1$-$C_4$, alcényloxy en $C_2$-$C_4$, alcynyloxy en $C_2$-$C_4$, haloalkyle en $C_1$-$C_4$, haloalcoxy en $C_1$-$C_4$, thioalkyle en $C_1$-$C_4$, hydroxyalkyle en $C_1$-$C_4$, alcoxy en $C_1$-$C_4$ alkyle en $C_1$-$C_4$, cycloalkyle en $C_3$-$C_6$, cycloalkyl en $C_3$-$C_6$ alkyle en $C_1$-$C_4$,

méthylènedioxy éventuellement substitué, aryle éventuellement substitué, hétéroaryle éventuellement substitué, hétérocyclyle éventuellement substitué, aryloxy éventuellement substitué, hétéroaryloxy éventuellement substitué, arylalkyle en $C_1$-$C_4$ éventuellement substitué dans lequel le segment alkyle est éventuellement substitué par un hydroxy, hétéroarylalkyle en $C_1$- $C_4$ éventuellement substitué, arylalcényle en $C_2$-$C_4$ éventuellement substitué, hétéroarylalcényle en $C_2$-$C_4$ éventuellement substitué, arylalcoxy en $C_1$- $C_4$ éventuellement substitué, hétéroarylalcoxy en $C_1$- $C_4$ éventuellement substitué, aryloxy- alkyle en $C_1$- $C_4$ éventuellement substitué, hétéroaryloxyalkyle en $C_1$- $C_4$ éventuellement substitué, acyloxy, cyano, thiocyano, nitro, -NR'R'', -NHCOR', -NHCONR'R'', -CONR'R'', -COOR', $-OSO_2R'$, $-SO_2R'$, -COR', -CR'=NR'' ou -N=CR'R'' dans lesquels R' et R'' sont indépendamment l'hydrogène, un groupe alkyle en $C_1$-$C_4$, alcoxy en $C_1$-$C_4$, thioalkyle en $C_1$-$C_4$, cycloalkyle en $C_3$-$C_6$, cycloalkyl en $C_3$-$C_6$ alkyle en $C_1$-$C_4$, phényle ou benzyle, les groupes phényle et benzyle étant éventuellement substitués par un halogène, un groupe alkyle en $C_1$-$C_4$ ou alcoxy en $C_1$-$C_4$ ; quand R' et R'' sont dans CONR'R'' ils peuvent former ensemble un noyau hétérocyclique à 5 ou 6 atomes ; ou deux substituants, quand ils sont en positions adjacentes sur le noyau aryle ou hétéroaryle pouvant se joindre pour former un noyau aliphatique fusionné; et $R^3$ et $R^4$ sont indépendamment l'hydrogène ou un groupe alkyle en $C_1$-$C_4$.

3. Composé selon la revendication 1 ou 2 dans lequel W est $CH_3O.CH=CCO_2CH_3$.

4. Composé selon la revendication 1, 2 ou 3 dans lequel $R^1$ est l'hydrogène ou un groupe alkyle en $C_1$-$C_4$.

5. Composé selon la revendication 1, 2 ou 3 dans lequel $R^2$ est le groupe méthyle.

6. Composé selon la revendication 1, 3, 4 ou 5, ayant la formule (Ib)

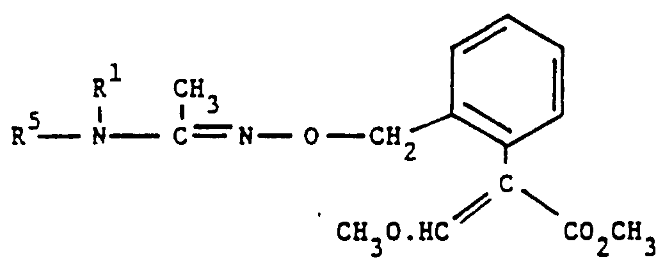

(Ib)

dans laquelle $R^1$ est l'hydrogène et R5 est un groupe phényle éventuellement substitué par un halogène, un groupe alkyle en $C_1$-$C_4$ ; alcényle $C_2$-$C_4$, alcynyle en $C_2$-$C_4$, alcoxy en $C_1$-$C_4$, alcényloxy en $C_2$-$C_4$, alcynyloxy en $C_2$-$C_4$, haloalkyle en $C_1$-$C_4$, haloalcoxy en $C_1$-$C_4$, alcoxy (en $C_1$-$C_4$) alkyle en $C_1$-$C_4$, acyloxy, cyano, thiocyano, nitro, -NR'R'', -NHCOR', -CONR'R'', -COOR' ou -COR', dans lesquels R' et R'' sont indépendamment l'hydrogène, un groupe alkyle en $C_1$-$C_4$ ou alcoxy en $C_1$-$C_4$.

7. Composé selon la revendication 6 dans lequel $R^1$ est l'hydrogène et $R^5$ est un groupe phényle éventuellement substitué par un halogène, un groupe cyano, nitro, haloalkyle en $C_1$-$C_4$ ou -COOR' dans lequel R' est un groupe alkyle en $C_1$-$C_4$.

8. Procédé de préparation d'un composé selon la revendication 1, le procédé comprenant la réaction d'un composé de formule (II) :

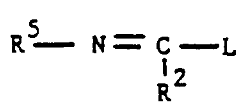

(II)

avec un composé de formule (III) :

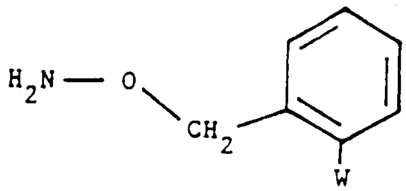

(III)

et éventuellement la réaction du produit formé avec un composé de formule $R^1L$ (dans laquelle $R^1$ est autre que l'hydrogène) ;

9. Composition fongicide comprenant une quantité efficace fongicide d'un composé selon la revendication 1 et un support ou un diluant de celui-ci acceptable du point de vue fongicide.

10. Procédé pour combattre les champignons qui comprend l'application à des plantes, à des graines de plantes ou à des endroits où sont situés les plantes ou les graines, d'un composé selon la revendication 1 ou une composition selon la revendication 9.

11. Composition insecticide comprenant une quantité efficace insecticide ou acaricide d'un composé selon la revendication 1 et un support ou un diluant de celui-ci acceptable du point de vue insecticide ou acaricide.

12. Procédé pour combattre des insectes ou acariens nuisibles qui comprend l'application à l'endroit où se trouvent les nuisibles d'une quantité efficace d'un composé selon la revendication 1 ou d'une composition selon la revendication 11.